# EUROPEAN PATENT APPLICATION

(11) **EP 1 855 112 A1**
(43) Date of publication of application: **14.11.2007**
(21) Application number: 06300450.1
(22) Date of filing: 10.05.2006
(51) Int. Cl.: G01N 33/68, C07K 14/16, G01N 33/569

(54) **Method for the in vitro screening of compounds inhibiting production of infectious HIV-1 virions**

(71) Applicant: Inserm, 75654 Paris Cedex 13 (FR)
(72) Inventor: Berlioz-Torrent, Clarisse, 75016 Paris (FR); Benarous, Richard, 75013 Paris (FR); Lopez-Verges, Sandra, 75011 Paris (FR)
(74) Representative: Michelet, Alain

(57) **Abstract**

Method for the *in vitro* screening of compounds that inhibit Env glycoprotein incorporation into HIV-1 virions in cells infected by a HIV-1 virus, wherein said method comprises the following steps
a) determining the ability of a candidate compound to inhibit the interaction between (i) the HIV-1 Gag protein and (ii) the TIP47 protein; and
b) selecting positively the candidate compound(s) that inhibit the interaction between (i) the HIV-1 Gag protein and (ii) the TIP47 protein.

## Description

### FIELD OF THE INVENTION

The present invention relates to the field of *in vitro* assays for the screening of therapeutically useful substances against infection with HIV-1 viruses.

### BACKGROUND OF THE INVENTION

AIDS disease, which is primarily caused by infection of individuals with a HIV retrovirus, is now the most devastating disease in the whole world, since the number of individuals which are, to date, infected with HIV viruses is estimated to about 40 millions of individuals.

During the sole year 2001, 5 millions of individuals were infected with HIV while 3 millions of individuals have deceased in the same time. Since the discovery of the AIDS causative agent in 1983, the HIV virus, extensive efforts have been made in order to understand the mechanism of action of this virus and to develop accurate methods for (i) reproducibly diagnosing the infection, as well as (ii) carrying out a prognosis of the progression of the disease in a given patient. When AIDS first surfaced in the United States, there were no medicines to combat the underlying immune deficiency and few treatments existed for the opportunistic diseases that resulted. During the past 10 years, however, researchers have developed drugs to fight both HIV infection and its associated infections and cancers. The U.S. Food and Drug Administration (FDA) has approved a number of drugs for treating HIV infection. The first group of drugs used to treat HIV infection, called nucleoside reverse transcriptase (RT) inhibitors, interrupts an early stage of the virus making copies of itself. Included in this class of drugs (called nucleoside analogs) are AZT, ddC (zalcitabine), ddl (dideoxyinosine), d4T (stavudine), 3TC (lamivudine), abacavir (ziagen), and tenofovir (viread). These drugs may slow the spread of HIV in the body and delay the start of opportunistic infections. Health care providers can prescribe non-nucleoside reverse transcriptase inhibitors (NNRTIs), such as delvaridine (Rescriptor), nevirapine (Viramune), and efravirenz (Sustiva), in combination with other antiretroviral drugs. More recently, FDA has approved a second class of drugs for treating HIV infection. These drugs, called protease inhibitors, interrupt virus replication at a later step in its life cycle. They include Ritonavir (Norvir), Saquinivir (Invirase), etc.

Because HIV can become resistant to any of these drugs, there is a need in the art for novel compounds of pharmaceutical interest that are biologically active in HIV-infected patients. Today, health care providers must use a combination treatment to effectively suppress the virus. When RT inhibitors and protease inhibitors are used in combination, it is referred to as highly active antiretroviral therapy, or HAART, and can be used by people who are newly infected with HIV as well as people with AIDS. Researchers have credited HAART as being a major factor in significantly reducing the number of deaths from AIDS in this country. While HAART is not a cure for AIDS, it has greatly improved the health of many people with AIDS and it reduces the amount of virus circulating in the blood to nearly undetectable levels. Researchers, however, have shown that HIV remains present in hiding places, such as the lymph nodes, brain, testes, and retina of the eye, even in patients who have been treated.

There is a need in the art for novel therapeutically useful compounds for preventing individuals from the occurrence of AIDS upon infection with a HIV virus or, more generally, for treating patients infected with a HIV virus. Particularly, in the definition of HAART, there is a need to include novel pharmaceutically active compounds that will specifically be directed against other target molecules than the HIV protease and the HIV retrotranscriptase and which will act on targets involved in distinct stages of the disease.

Despite the beneficial effects of HAART, there are side effects associated with the use of antiviral drugs that can be severe. Some of the nucleoside RT inhibitors may cause a decrease of red or white blood cells, especially when taken in the later stages of the disease. Some may also cause inflammation of the pancreas and painful nerve damage. There have been reports of complications and other severe reactions, including death, to some of the antiretroviral nucleoside analogs when used alone or in combination. Therefore, health care experts recommend that people on antiretroviral therapy be routinely seen and followed by their health care providers. The most common side effects associated with protease inhibitors include nausea, diarrhea, and other gastrointestinal symptoms. In addition, protease inhibitors can interact with other drugs resulting in serious side effects. In front of the problems bound to the adverse effects of HAART there is still a need in the art for novel therapeutically useful compounds, which would be deprived, at least substantially, of adverse effects.

### SUMMARY OF THE INVENTION

The present invention relates to various *in vitro* assays for the screening of compounds that inhibit HIV-1 Env glycoprotein incorporation into HIV-1 virions.

More precisely, this invention pertains to *in vitro* assays for the screening of compounds that interfere with the binding of the MA domain of the HIV-1 Gag protein to the cellular TIP47 protein.

Thus, an object of this invention consists of a method for the screening of compounds that inhibit Env glycoprotein incorporation into HIV-1 virions in cells infected by a HIV-1 virus, wherein said method comprises the following steps
a) determining the ability of a candidate compound to inhibit the interaction between (i) the HIV-1 Gag protein and (ii) the TIP47 protein; and
b) selecting positively the candidate compound(s) that inhibit the interaction between (i) the HIV-1 Gag protein and (ii) the TIP47 protein.

Another object of the present invention consists of a method for the screening of compounds that inhibit formation of infectious HIV-1 virions within cells infected with HIV-1 virus, wherein said method comprises the steps of :
i) screening for compounds that inhibit Env glycoprotein incorporation into HIV-1 virions in cells infected by a HIV-1 virus, by performing the screening method defined above; and
ii) screening the compounds positively selected at the end of step i) for their ability to inhibit formation of infectious HIV-1 virions within cells infected with HIV-1 virus.

This invention also pertains to anti-HIV-1 compounds that have been positively selected by performing any one of the *in vitro* screening methods described herein, as well as to pharmaceutical compositions comprising the said positively selected compounds.

More specifically, this invention pertains to an anti-HIV-1 compound consisting of a polypeptide comprising the amino acid sequence of SEQ ID N°1, as well as to pharmaceutical compositions containing the same.

### DESCRIPTION OF THE FIGURES

### Figure 1 - TIP47 depletion decreases HIV-1 replication.

**Fig 1A. siRNA targeted against TlP47 decreases HIV-1 replication in HeLa P4-2 cells.** HeLa P4-2 cells were transfected twice with Luc or TIP-A or TIP-B siRNAs and infected with HIV-1 HXB2R virus. The amount of p24 in cell supernatants was quantified 96h after infection using ELISA. The expression of TIP47 and tubulin was analysed by western blotting with anti-TlP47 and anti-tubulin. The data are representative of five independent experiments. NI: non-infected cells.

**Fig 1B. siRNA targeted against TlP47 have no effect on the entry steps of infection.** CD4⁺ HeLa P4-2 indicator cells were transfected twice with Luc or TIP-A or TIP-B siRNA and infected with HXB2R HIV-1 (100 ng of p24). Then, the cells were placed in medium containing AMD3100 (a CXCR4 inhibitor). The following day, the cells were assayed for β-gal activity. The number of infected cells was determined by counting the number blue cells indicating β-gal activity. The data are representative of three independent experiments. Nl: non-infected cells.

### Figure 2 - TIP47 depletion decreases Env incorporation.

**Fig 2A.** HeLa cells were transfected twice with the HIV-1 HXB2R proviral DNA and pAS1B-HA-TIP47 (lane 5) or pASIB vectors (lanes 2-4), and 30 nM of indicated siRNA. The cells and virion lysates were subjected to western blotting with anti-p24 (ARP366), anti-TMgp41 (2F5), rabbit anti-TIP47 and anti-tubulin antibodies 48h after transfection.

**Fig 2B.** p24 Gag antigen production in supernatants was quantified by ELISA.

**Fig 2C.** Supernatants were used to infect CD4⁺ HeLa P4-2 indicator cells to determine the titer of the produced virus. HIV-1 infectivity corresponded to the ratio between the titer of the produced virus and the quantity of p24 detected in the supernatant. These data are representative of three independent experiments. NT: non-transfected.

### Figure 3 - Overexpression of TIP47 enhances Env incorporation.

Cell (upper panel) and virion lysates (lower panel) were prepared from HeLa cells transfected with pcDNA3 (lane 1) or pAS1B-HA-TIP47 (lane 3) and wt HIV1 HXB2 proviral DNA (lanes 1, 3). The amount of Gag (p55Gag, p41, CAp24) and Env (TMgp41) protein was analysed by western blotting with either an anti-CAp24 or an anti-TMgp41 or an anti-HA antibodies. These results are representative of experiments performed in triplicate. NT: non-transfected cells.

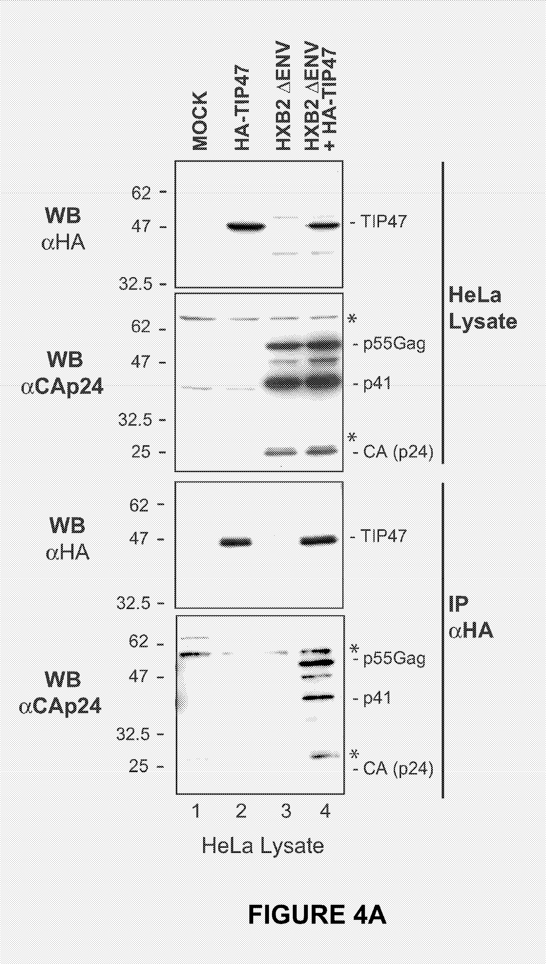

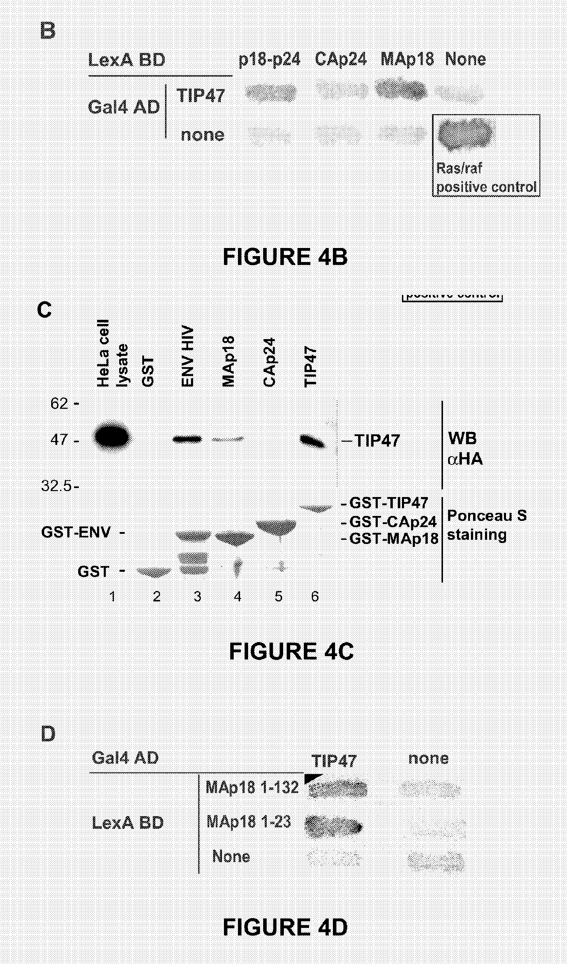

### Figure 4 - interaction between MAp18 and TIP47.

### Fig 4A. Co-immunoprecipitation between Gag proteins and

**TIP47.** HeLa cells were transfected with pcDNA3 (lane 1) or pcDNA3 and pAS1B-HA-TIP47 (lane 2) or pcDNA3 and HXB2R ΔENV proviral DNA (lane 3) or pAS1B-HA-TIP47 and HXB2R ΔENV proviral DNA (lane 4). HeLa cell lysates were precipitated with anti-HA 12CA5 mAb. Crude lysates (upper panel) and the precipitated proteins (lower panel) were detected with rabbit anti-CAp24 and anti-HA 3F10 HRP mAbs. The nonspecific bands are marked with an asterisk.

**Fig 4B. Interaction of MAp18 with TIP47 in yeast two-hybrid assay.** The yeast reporter strain L40 producing the hybrid proteins indicated was analysed for β-galactosidase expression. Fragments corresponding to MAp18, CAp24 and MAp18-CAp24 were fused to the N-terminus of the lexA-binding domain (lexA BD), and the TIP47 ORF was fused to the Gal4 activation domain (Gal4 AD). Ras and Raf proteins, which efficiently bind to each other, were used as a positive control.

**Fig 4C. Binding of the MAp18 to TIP47 in HeLa cell lysate binding assay.** HeLa cell lysates producing HA-TIP47 were incubated with equal amounts (5 µg) of purified GST (lane 2) or GST fused to either the cytoplasmic domain of HIV-1 TMgp41 (ENV HIV) (lane 3), MAp18 (lane 4), CAp24 (lane 5) or TIP47 (lane 6). TIP47 binding was analysed by western blotting with an anti-HA mAb. Crude lysates corresponding to 2 x 10⁵ cells were run as a control (lane 1).

**Fig 4D. Interaction of fragment 1-23 of MAp18 with TIP47 in a yeast two-hybrid assay.** The yeast reporter strain L40 producing the hybrid proteins indicated was analysed for β-galactosidase expression. The amino acids residues 1-23 (MAp18 1-23) or 1-132 (MAp18 1-132, corresponding to the full length MA domain) of the MA domain were fused to the N-terminus of the lexA-binding domain (lexA BD), and the TIP47 ORF was fused to the Gal4 activation domain (Gal4 AD).

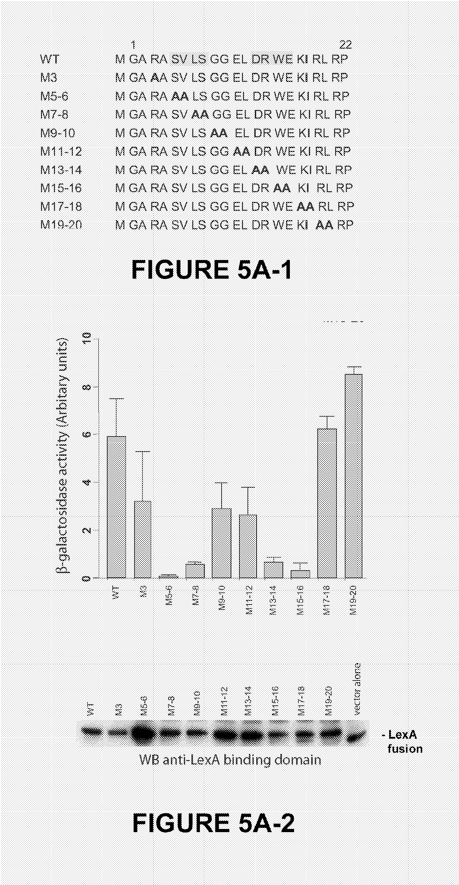

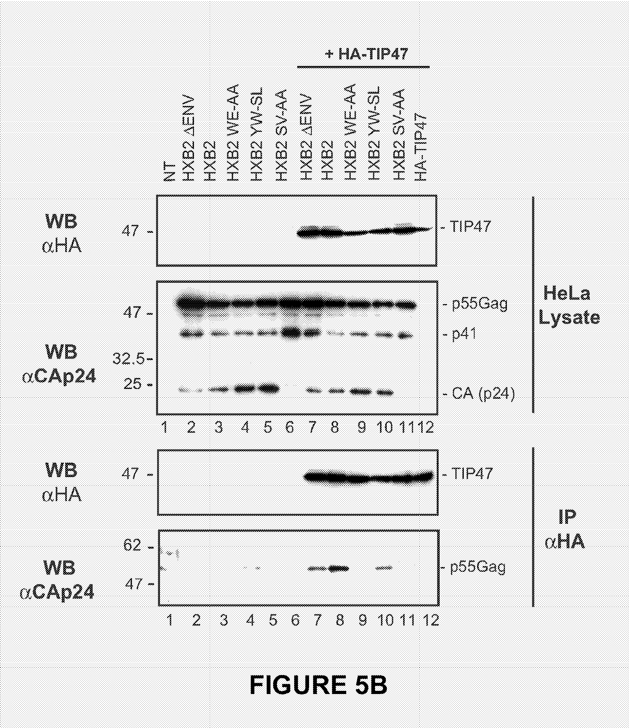

### Figure 5 - The amino acid residues 5 to 16 of the matrix are required for binding to TIP47.

**Fig 5A. Mapping of the region involved in binding of TIP47 by quantitative two-hybrid assays.** The mutated FBL-MA₁₋₂₃ vectors generated by PCR-directed mutagenesis are shown (Fig. 5A-1). Yeast L40 reporter strains containing the β̃ *Ga*l*L*exA-inducible gene were co-transformed and the interaction between MA and TIP47 was evaluated by β-gal activity in a liquid culture assay. Experiments were performed in duplicate and the results are representative of three independent experiments. Expression of the MA-LexA BD fusion protein was checked by western blotting with an anti-LexA antibody. (Fig. 5A-2).

**Fig 5B - Co-immunoprecipitation between MA mutants and TIP47.** HeLa cells were transfected with pcDNA3 (lanes 1-6) or pAS1B-HA-TIP47 (lanes 7-12) and either HXB2R ΔENV (lanes 2,7), HXB2R wt (lanes 3,8), HXB2R W₁₅E₁₆-AA (lanes 4,9), HXB2R Y₈₀₂W₈₀₃-SL (lanes 5,10), HXB2R S₅V₆-AA (lanes 6,11) proviral DNAs or pcDNA3 (lanes 1,12). HeLa cell lysates were precipitated with anti-HA 12CA5 mAbs. Crude lysates (upper panel) and precipitated proteins (lower panel) were detected with rabbit anti-CAp24 and anti-HA 3F10 HRP mAbs.

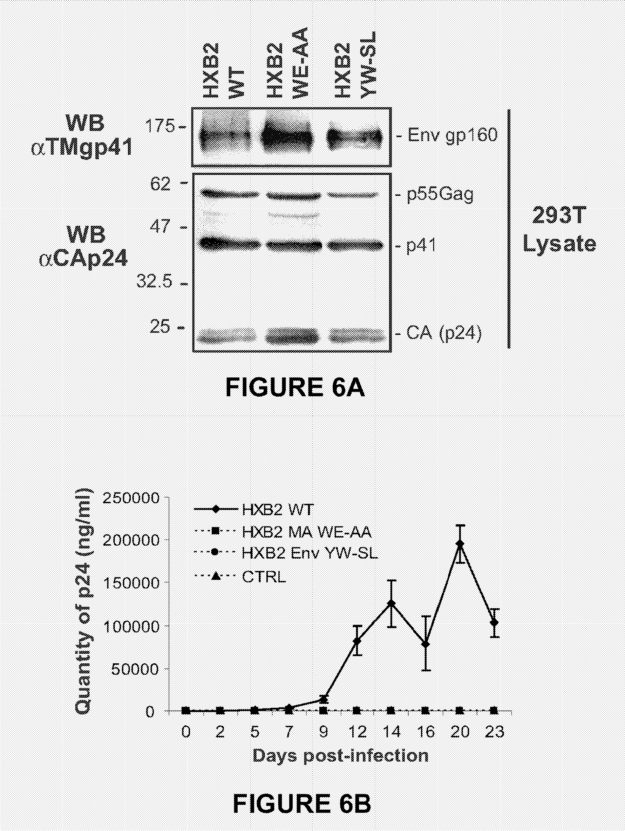

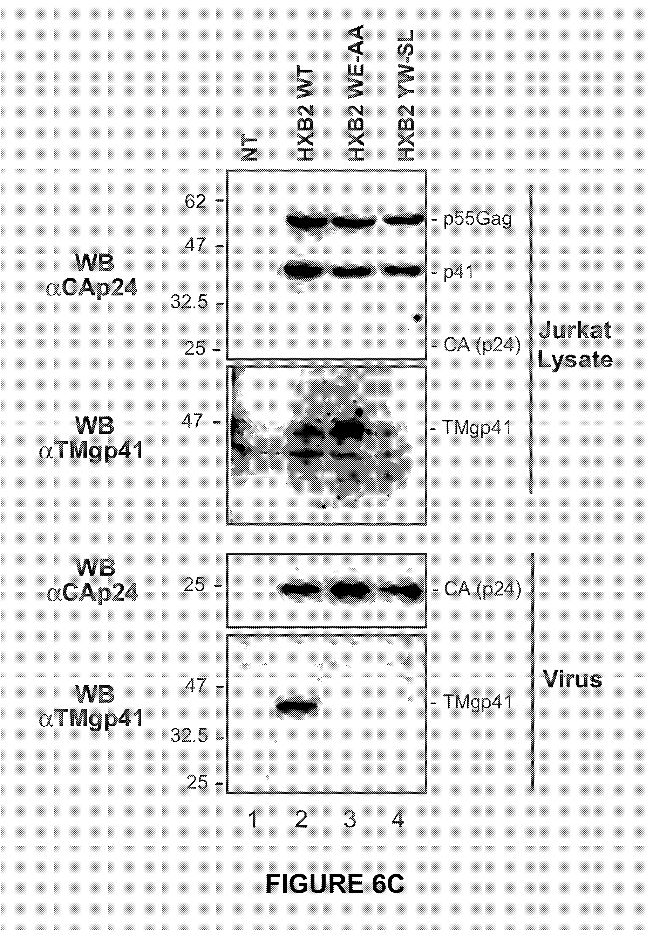

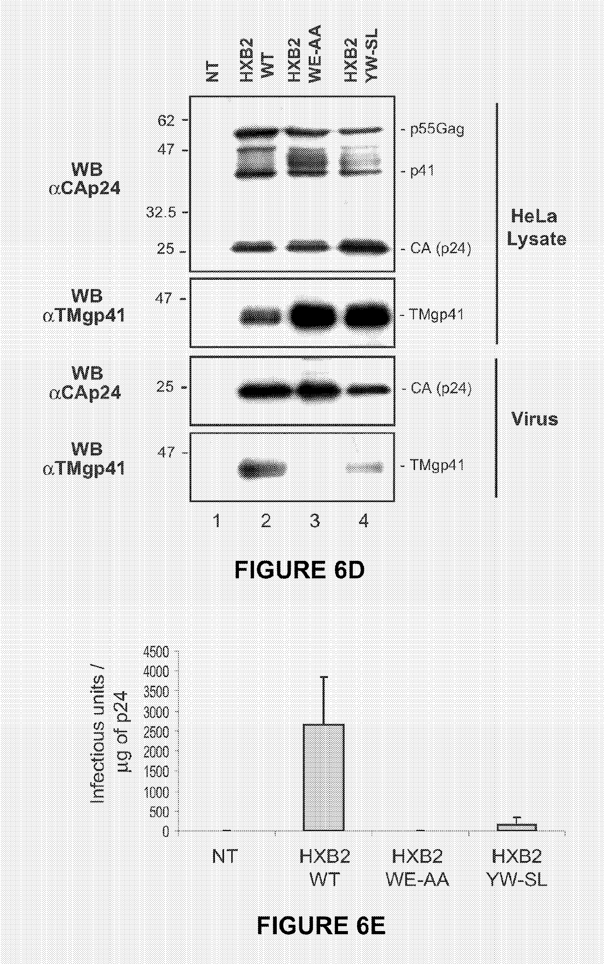

### Figure 6 - Replication and Env incorporation in Jurkat T cells and HeLa cells of wt HIV-1 and MA W₁₅E₁₆-AA and Env Y₈₀₂W₈₀₃-SL mutant HIV-1.

**Fig 6A.** Gag (p55Gag, CAp24) and Env (Envgp160) content of 293T cell lysates transfected with HIV-1 HXB2 wt, and MA W₁₅E₁₆-AA and Env Y₈₀₂W₈₀₃-SL proviral DNA mutants were analysed by western blotting with anti-TMgp41 and anti-CAp24 Abs.

**Fig 6B. Replication kinetics.** Jurkat T-cells were infected in parallel with HIV-1 HXB2R wt, and MA W₁₅E₁₆-AA and Env Y₈₀₂W₈₀₃-SL mutant HIV-1 (50 ng of p24 / 10⁶ cells). Every two to three days, culture supernatant samples were collected for p24 antigen quantification. Values are the averages of three independent experiments ± SD. CTRL: non-infected cells.

**Fig 6C. Analysis of Env incorporation into wt, MA W₁₅E₁₆-AA and Env Y₈₀₂W₈₀₃-SL mutant virions in Jurkat T cells.** Cell (upper panel) and virion (lower panel) lysates were prepared from Jurkat T cells infected with wt, MA W₁₅E₁₆-AA and Env Y₈₀₂W₈₀₃-SL HIV1 virus stocks pseudotyped with VSV-G. The amount of Gag (p55Gag, CAp24) and Env (TMgp41) proteins was analysed by western blotting with either anti-p24 (CA) or anti-TMgp41 mAbs. The results are representative of duplicate experiments. NT: non-transfected cells.

**Fig 6D. Analysis of Env incorporation into wt, MA W₁₅E₁₆-AA and Env Y₈₀₂W₈₀₃-SL mutant virions in HeLa cells.** HeLa cells were transfected with HIV1 HXB2R wt or MA W₁₅E₁₆-AA or Env Y₈₀₂W₈₀₃-SL mutant provirus. Cell and virion lysates were analysed as described above. NT: non-transfected cells.

**Fig 6E. Infectivities of wt HIV-1 HXB2R, and MA W₁₅E₁₆-AA or Env Y₈₀₂W₈₀₃-SL mutants.** Supernatants containing viruses from HeLa cells were normalized for p24 activity and then used to infect the HeLa P4-2 indicator cell line. The β-galactosidase activities of infected P4-2 cells were measured. The data are representative of three independent experiments.

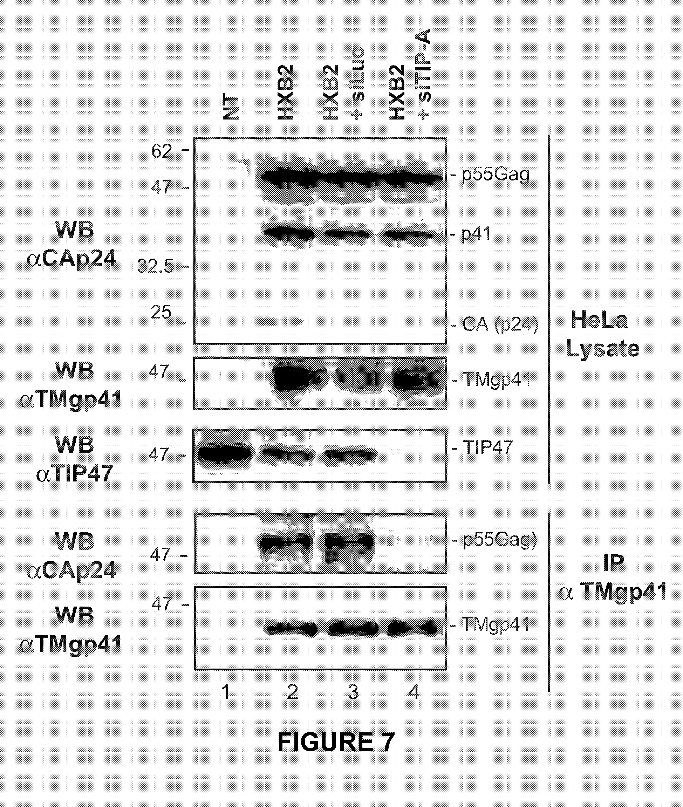

**Figure 7 - Reduction of Gag-Env co-immunoprecipitation by TlP47 silencing.** HeLa P4-2 cells were transfected twice with HIV1 HXB2R proviral DNA and TIP-A siRNA (lane 4) or control (Luc, lane 3) siRNA. HeLa cell lysates were precipitated with anti-TMgp41 (41A) mAb. Crude lysates (upper panel) and the precipitated proteins (lower panel) were detected with rabbit anti-CAp24, anti-TMgp41 2F5 and rabbit anti-TIP47 antibodies.

### DETAILED DESCRIPTION OF THE INVENTION

Surprisingly, it has been found according to the invention that a specific protein domain of the HIV-1 Gag protein, namely the MA domain, had the ability to bind to the TIP47 cellular protein, in cells infected by a HIV-1 virus.

It has been further unexpectedly found that the binding of the MA domain of the HIV-1 Gag protein to TIP47 induces the incorporation of the HIV-1 Env glycoprotein into the HIV-1 virions under formation.

Further, it has been shown according to the invention that the Env glycoprotein incorporation into the virions under formation was blocked by inhibiting the binding between the MA domain of the Gag protein and TIP47

Still further, it has been found that preventing the binding between the MA domain of the Gag protein and TIP47 within cells infected with a HIV-1 virus leads, at most, to the production of non-infectious virions.

Yet further, it has been shown according to the invention that the cellular TIP47 protein, which was already known in the art to bind to the Env glycoprotein, acts as a connector between the Env glycoprotein and the Gag protein, and that TlP47 binds independently to Env and Gag, respectively.

More precisely, it has been shown according to the invention that the expression of TIP47 is essential in producer cells for incorporating the envelope glycoprotein in HIV-1 Gag particles to confer full infectivity to budding HIV-1 viral particles. This is supported in the examples herein that disclose :
i) the silencing of TIP47 by specific siRNAs leads to impaired Env incorporation in viral particles released into the supernatant of the producer cells. This results in a non-infectious virus;
ii) the back complementation of the siRNA treatment by expression of a TIP47 construct resistant to siRNA restored both Env incorporation and virion infectivity to similar levels as observed in virions released from normal cells; and
iii) the overexpression of TIP47 caused a significant increase in Env incorporation.

The molecular mechanism of TIP47 involvement in Env incorporation has also been determined herein. TIP47 binds to the cytoplasmic tail of TMgp41 and to the MA domain of Gag, thus connecting these two essential virus components to ensure that the envelope glycoprotein is packaged into Gag particles. This is supported in the examples herein that disclose :
i) that TIP47 interacts with both MA and the cytoplasmic tail of TMgp41;
ii) that the association of Env with Gag is dependent on TIP47 expression. Indeed, depletion of TIP47 by siRNA treatment impaired the association of Env and Gag in a co-immunoprecipitation assay;
iii) that Gag is still associated with TIP47 in cells that produce a virus harbouring an Env Y₈₀₂W₈₀₃-SL mutation that is unable to bind to TIP47. This shows that TIP47 binds independently to Env and Gag; and
iv) that mutations in essential amino acid residues in the MA domain (W₁₅E₁₆-AA) or in the cytoplasmic tail of TMgp41 (Y₈₀₂W₈₀₃-SL) resulting in TIP not binding to either of these viral proteins lead to impaired Env incorporation and, thus, non-infectious viruses. Altogether, these results show that Env incorporation requires TIP47 to associate with both the MA domain and the TMgp41 Env subunit, thus connecting Gag and Env.

Thus, it has been found according to the present invention that any compound or substance having the ability to prevent the binding between the HIV-1 Gag protein and TIP47 is potentially useful as an active ingredient for preventing or treating a disease linked to the infection of an individual with a virus of the HIV family. Such inhibitor compound or substance inhibits or blocks the fully infectious HIV-1 virus production in individuals infected with an HIV-1 virus.

As intended herein, a HIV-1 virus which is not fully infectious consists of a HIV-1 viral particle possessing reduced ability to bind to target cells and/or reduced ability to infect target cells, as compared with wild-type HIV-1 viral particles such as those encoded by the wt HIV1 HXB2 proviral DNA. According to the invention, a HIV1 viral particle having reduced infectious properties encompass HIV1 viral particles having an ability to infect target cells of less than 50% the ability of wild-type HIV1 viral particles to infect the same target cells. Assessment of the ability of given HIV1 viral particles to infect target cells may be performed according to conventional techniques well known in the art. Illustratively, the ability of HIV1 viral particles to infect target cells may be assessed by (i) bringing into contact cultured target cells with the HIV1 viral particles to be tested and then (ii) quantifying the HIV1 p24 protein in the culture supernatant. Further illustratively, the ability of HIV1 viral particles to infect target cells may be assessed by (i) bringing into contact cultured target cells with the HIV1 viral particles to be tested and (ii) testing the infectiosity of the viral particles produced by the infected target cells on indicator cells such as the HeLa P4-2 cell line. These two techniques are fully described in the examples herein.

Mainly as intended herein, as it is shown in the examples, a HIV-1 virus which is not fully infectious consists of HIV-1 viral particles the infectiosity of which is reduced due to an impaired Env incorporation.

Anti-HIV-1 compounds or substances according to the invention, which inhibit or block the fully infectious HIV-1 virus production in individuals infected with an HIV-1 virus, may be screened by any screening method comprising a step of detecting a reduced binding, or the lack of binding, between (i) the Gag protein, or a suitable peptide fragment thereof and (ii) the TIP47 protein, or a suitable peptide fragment thereof.

Thus, an object of the present invention consists of a method for the *in vitro* screening of compounds that inhibit Env glycoprotein incorporation into HIV-1 virions in cells infected by a HIV-1 virus, wherein said method comprises the following steps
a) determining the ability of a candidate compound to inhibit the interaction between (i) the HIV-1 Gag protein and (ii) the TIP47 protein; and
b) selecting positively the candidate compound(s) that inhibit the interaction between (i) the HIV-1 Gag protein and (ii) the TIP47 protein.

At step a), any method suitable for the screening of protein-protein interactions is suitable.

In almost all embodiments of step a), if not in whole embodiments of step a), the said step comprises a first step a1) consisting of assaying, directly or indirectly, for the ability of binding of the HIV-1 Gag protein to the cellular TIP47 protein in the presence of the candidate compound to be tested and wherein physical values illustrating the level of binding between these two proteins are obtained, and a second step a2) wherein the physical values obtained at step a1) are compared with normal or standard physical values obtained in the same assay performed in the absence of the said candidate compound. If, after comparison of the assay physical values with the normal or standard physical values, it is determined that the said candidate compound alters the binding between HIV-1 Gag and TIP47, then the said candidate compound is positively selected, at step b) of the screening method above.

As it will be fully illustrated further in the present specification, the "physical values" that are referred to above may be of various kinds, depending of the type of binding assay that is performed at step a) or a1). Notably, those physical values encompass light absorbance values (D.O.), radioactive signal values and intensity value of a fluorescence signal.

As it will be further detailed hereunder, not all embodiments of step a) or a1) of the screening method above require that (i) HIV-1 Gag, or a peptide fragment thereof, and (ii) TlP47 or a peptide fragment thereof be both included in the assay performed. Rather, in some embodiments of the screening method above, step a1) may consist of an assay for the binding of the candidate compound to be tested to either (i) the HIV-1 Gag, or a peptide fragment thereof, or (ii) TIP47 or a peptide fragment thereof. Indeed, in most cases, if not in all cases, the binding of the said candidate compound to either (i) the HIV-1 Gag, or a peptide fragment thereof, or (ii) TIP47 or a peptide fragment thereof induces that the said candidate compound also interferes with the binding together of the HIV-1 Gag protein to TIP47.

### Preferred embodiments of the screening method using either Gag, TIP47, or peptide fragments thereof.

As it is readily understood from the experimental results described herein, compounds that inhibit the interaction between (i) the HIV-1 Gag protein and (ii) the TIP47 protein encompass those compounds that bind either to the HIV-1 Gag protein or to TIP47, provided that the binding of the said compounds of interest then prevents the binding of TIP47 or HIV-1 Gag, respectively, to its partner protein.

Thus, in certain embodiments of step a) a method for the screening of compounds that bind to either the HIV-1 Gag protein or to TIP47 is suitable.

In certain other embodiments of the method above, step a) consists of a screening method comprising a step of detecting the alteration of the HIV-1 Gag protein/TIP47 protein-protein interactions that is induced by a candidate compound under testing.

Whatever the embodiment of step a) of the screening method above, the complete HIV-1 Gag protein and the complete TIP47 protein may be used as the binding partners.

However, it has been found according to the present invention that the HIV-1Gag protein and TIP47 bind via specific protein regions of each complete protein. For optimal accuracy of the screening method, including optimal specificity of the binding events that are detected, as well as for financial costs incurred by performing the screening method above, using suitable polypeptide fragments of the HIV-1 Gag protein and/or of TIP47 is preferable.

As already mentioned above, it has been found according to the invention that TIP47 binds specifically to the MA domain of HIV-1 Gag.

More precisely, it has been found that TIP47 binds to a small peptide fragment of 12 amino acid residues in length which is located close to the N-terminal end of HIV-1 Gag. Said peptide fragment is described herein as the amino acid sequence of SEQ ID N° 1. The amino acid sequence of said peptide fragment has been found to be variable at specific amino acid positions in the various HIV-1 isolates and thus TIP47 binds to various peptide members of the family of peptide fragments having one or more amino acid substitutions as compared to SEQ ID N° 1. The said family of peptide fragments which consist of the minimal peptides originating from a HIV-1 Gag protein that bind to TIP47 are selected from the group consisting of :
(A) the peptide consisting the amino acid sequence of SEQ ID N° 6 below :
   NH₂-S-X₄-L-X₅-X₆-X₇-X₈-X₉-X₁₀-X₁₁-W-E- COOH, wherein
   - X4 is an amino acid residue selected from the group consisting of V, L, I, T, R and E;
   - X5 is an amino acid residue selected from the group consisting of S, T, R, I and K;
   - X6 is an amino acid residue selected from the group consisting of G and A;
   - X7 is an amino acid residue selected from the group consisting of G, R, K, E, S, T, K and D;
   - X8 is an amino acid residue selected from the group consisting of E, K, R, Q and G;
   - X9 is an amino acid residue selected from the group consisting of L, F, K and Q;
   - X10 is an amino acid residue selected from the group consisting of D and E; and
   - X11 is an amino acid residue selected from the group consisting of R,A,S,K,Q,T,E,N,D
(B) the peptide consisting the amino acid sequence of SEQ ID N° 7 below :
   NH₂-X₁-X₂-A-X₃-A-S-X₄-L-X₅-X₆-X₇-X₈-X₉-X₁₀-X₁₁-W-E-X₁₂-X₁₃-X₁₄-X₁₅-X₁₆-P-COOH, wherein
   - X1 is an amino acid residue selected from the group consisting of M, L, V and I;
   - X2 is an amino acid residue selected from the group consisting of G, S and T;
   - X3 is an amino acid residue selected from the group consisting of R, G, S and K;
   - X4 is an amino acid residue selected from the group consisting of V, L, I, T, R and E;
   - X5 is an amino acid residue selected from the group consisting of S, T, R, I and K;
   - X6 is an amino acid residue selected from the group consisting of G and A;
   - X7 is an amino acid residue selected from the group consisting of G, R, K, E, S, T, K and D;
   - X8 is an amino acid residue selected from the group consisting of E, K, R, Q and G;
   - X9 is an amino acid residue selected from the group consisting of L, F, K and Q;
   - X10 is an amino acid residue selected from the group consisting of D and E;
   - X11 is selected from the group consisting of R, A, S, K, Q, T, E, N, D and P;
   - X12 is an amino acid residue selected from the group consisting of K, R, N, E, A and S;
   - X13 is an amino acid residue selected from the group consisting of I, M, Q and T;
   - X14 is an amino acid residue selected from the group consisting of R, K, W, Q, H and Y;
   - X15 is an amino acid residue selected from the group consisting of L and S; and
   - X16 is an amino acid residue selected from the group consisting of R and K.
   and
(C) a peptide having at least 60% amino acid identity with the Gag amino acid sequence of SEQ ID N°1.

Thus, at step a) of the screening method above, whatever the embodiment used, the only requirement, when using a HIV-1 Gag peptide fragment, is the presence in the said HIV-1 Gag peptide fragment of one of the HIV-1 Gag minimal peptide fragments defined above.

Further, it has been found that the HIV-1 Gag protein, or the minimal peptide fragment of SEQ ID N°1 derived thereof, binds to a specific protein region starting at the amino acid residue in position 200 and ending at the amino acid residue in position 333 of the TlP47 amino acid sequence of SEQ lD N° 2.

Thus, at step a) of the screening method above, whatever the embodiment used, the only requirement, when using a TIP47 peptide fragment, is the presence in the said TIP47 peptide fragment of at least a suitable portion of the said amino acid sequence starting at the amino acid residue in position 200 and ending at the amino acid residue in position 333 of the TIP47 amino acid sequence of SEQ ID N° 2.

In certain embodiments of the *in vitro* screening method above, step a) consists of the following steps :
a1) bringing into contact the candidate compound to be tested with a target polypeptide selected from the group consisting of:
   (1) a first polypeptide comprising the amino acid sequence of a minimal HIV-1 Gag minimal peptide fragment that is defined above;
      or
   (2) a second polypeptide comprising the amino acid sequence of SEQ ID N°2 or a peptide fragment thereof;
a2) determining the ability of said candidate compound to bind to said target polypeptide;

In the embodiments of step a) above, what is assayed is the binding of a candidate compound onto one binding partner selected from the group consisting of the HIV-1 Gag protein, or any suitable peptide fragment thereof, and TIP47, or any suitable peptide fragment thereof. It is taken into account that a candidate compound that binds to the suitable protein regions of either HIV-1 Gag or TIP47 necessarily alters the binding between HIV-1 Gag and TIP47.

Step a) of the embodiment above may be performed by using any method known in the art that allows detection of the binding of a compound of interest onto a protein material. These methods include immunological techniques including immunoassays, chromatography techniques including affinity chromatography, gel migration techniques including Western blotting techniques, biosensor techniques, as well as fluorescence techniques including those using FRET. Several of these techniques useful for the screening of binding events involving at least one protein binding partner will be disclosed in more details further in the present specification.

### Preferred embodiments of the binding partner derived from HIV-1 Gag

As already specified above, TIP47 binds to a very small protein region of the HIV-1 gag protein, the said small region being located within the Gag MA domain, at the N-terminal end of Gag.

Consequently, for performing step a) of the screening method above using a Gag peptide fragment as a binding partner, the main, or even the only, requirement consists of the use of a binding partner comprising the minimal peptide of Gag that binds to TIP47, which minimal peptide of Gag is selected from the group consisting of :
(A) the peptide consisting the amino acid sequence of SEQ lD N° 6 below :
   NH₂-S-X₄-L-X₅-X₆-X₇-X₈-X₉-X₁₀-X₁₁-W-E- COOH, wherein
   - X4 is an amino acid residue selected from the group consisting of V, L, I, T, R and E;
   - X5 is an amino acid residue selected from the group consisting of S, T, R, I and K;
   - X6 is an amino acid residue selected from the group consisting of G and A;
   - X7 is an amino acid residue selected from the group consisting of G, R, K, E, S, T, K and D;
   - X8 is an amino acid residue selected from the group consisting of E, K, R, Q and G;
   - X9 is an amino acid residue selected from the group consisting of L, F, K and Q;
   - X10 is an amino acid residue selected from the group consisting of D and E; and
   - X11 is an amino acid residue selected from the group consisting of R,A,S,K,Q,T,E,N,D
(B) the peptide consisting the amino acid sequence of SEQ ID N° 7 below :
   NH₂-X₁-X₂-A-X₃-A-S-X₄-L-X₅-X₆-X₇-X₈-X₉-X₁₀-X₁₁-W-E-X₁₂-X₁₃-X₁₄-X₁₅-X₁₆-P-COOH, wherein
   - X1 is an amino acid residue selected from the group consisting of M, L, V and I;
   - X2 is an amino acid residue selected from the group consisting of G, S and T;
   - X3 is an amino acid residue selected from the group consisting of R, G, S and K;
   - X4 is an amino acid residue selected from the group consisting of V, L, I, T, R and E;
   - X5 is an amino acid residue selected from the group consisting of S, T, R, I and K;
   - X6 is an amino acid residue selected from the group consisting of G and A;
   - X7 is an amino acid residue selected from the group consisting of G, R, K, E, S, T, K and D;
   - X8 is an amino acid residue selected from the group consisting of E, K, R, Q and G;
   - X9 is an amino acid residue selected from the group consisting of L, F, K and Q;
   - X10 is an amino acid residue selected from the group consisting of D and E;
   - X11 is selected from the group consisting of R, A, S, K, Q, T, E, N, D and P;
   - X12 is an amino acid residue selected from the group consisting of K, R, N, E, A and S;
   - X13 is an amino acid residue selected from the group consisting of I, M, Q and T;
   - X14 is an amino acid residue selected from the group consisting of R, K, W, Q, H and Y;
   - X15 is an amino acid residue selected from the group consisting of L and S; and
   - X16 is an amino acid residue selected from the group consisting of R and K.
      and
(C) a peptide having at least 60% amino acid identity with the Gag amino acid sequence of SEQ ID N°1.

The Gag peptide fragments defined above may also be termed herein a "Gag-derived" peptide sequences.

An illustrative embodiment of a Gag peptide fragment used as a binding partner in a screening method according to the invention consists of a peptide comprising the amino acid sequence of SEQ lD N°1.

As already explained herein, the inventors have shown that the general mechanism of HIV-1 viral particles production by HIV-1-infected cells include a crucial step of binding of the HIV-1 Gag protein to the cellular TIP47 protein, the said step being crucial for Env incorporation in the viral particles under formation. This crucial step for production of infectious HIV-1 viral particles will thus necessarily occur for all infectious HIV-1 isolates and is independent of infectious HIV-1 variability. Thus, the cellular TlP47 protein will bind to the Gag protein originating from every infectious HIV-1 isolate, and specifically to the said minimal MA domain-derived binding sequence from Gag, it being taken into account that the said minimal MA-derived binding sequence from infectious HIV-1 isolates encompass peptide sequences having at least 60% amino acid identity with the amino acid sequence of SEQ ID N° 1, as found according to the invention.

As intended herein, a peptide sequence having at least 60% amino acid identity with the reference peptide of SEQ ID N°1 possesses at least 60%, 65%, 70%, 75%, 80%, 85%, 90%, 91 %, 92%, 93%, 94%, 95%, 96%, 97%, 98%, 99% or 99.5% amino acid identity with said reference peptide of SEQ lD N°1.

"Percentage of sequence identity," as used herein, is determined by comparing two optimally aligned sequences over a comparison window, where the fragment of the polynucleotide or amino acid sequence in the comparison window may comprise additions or deletions (e.g., gaps or overhangs) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, Wis.), or by inspection. Given that two sequences have been identified for comparison, GAP and BESTFIT are preferably employed to determine their optimal alignment. Typically, the default values of 5.00 for gap weight and 0.30 for gap weight length are used.

For optimal comparison purposes, the percent of identity of two peptide sequences can be achieved with CLUSTAL W (version 1.82) with the following parameters: (1) CPU MODE = ClustalW mp; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

Thus, according to the invention, the said binding partner may be any polypeptide comprising a Gag-derived amino acid sequence as defined above, selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1.

Such polypeptide may comprise, additionally to a Gag-derived amino acid sequence, also further amino acid sequence(s), such as, for example a poly-histidine tail or the amino acid sequence of GST.

As used herein, an embodiment of a "Gag-derived" amino acid sequence consists of an amino acid sequence consisting of at least 16 consecutive amino acid residues of the Gag protein of SEQ ID N°3, which Gag-derived amino acid sequence always comprises an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1.

Indeed, these encompass polypeptides comprising the amino acid sequence of SEQ ID N° 1, including the polypeptide consisting of the amino acid sequence of SEQ ID N°1, that may be optionally labelled with a detectable molecule.

Polypeptides comprising the amino acid sequence of SEQ ID N°1 encompass also any HIV-1 Gag-derived peptide fragment comprising the said amino acid sequence of SEQ ID N°1, including the complete HIV-1 Gag protein of SEQ ID N°3.

The amino acid sequence of SEQ ID N°1 is comprised in the Gag protein sequence of SEQ ID N°3, from the Serine residue (also termed S or Ser) at position 6 to the Glutamic acid residue (also termed E or Glu) at position 17 of SEQ ID N°3.

Illustratively, polypeptides comprising the amino acid sequence of SEQ ID N°1 encompass polypeptides comprising at least 16 consecutive amino acid residues of SEQ ID N°3 and comprising the amino acid sequence of SEQ ID N°1.

Short length Gag-derived polypeptides of less than 400 amino acid residues in length, advantageously of less than 300 amino acid residues in length, more preferably of less than 200 amino acid residues in length and most preferably of less than 100 amino acid residues in length are preferred.

In certain embodiments, Gag-derived polypeptides comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1 encompass polypeptides comprising at least 16, 17,18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 amino acids in length.

In certain embodiments, Gag-derived polypeptides encompass polypeptides comprising at least 16, 17,18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 consecutive amino acid residues of SEQ lD N°3 and comprising the amino acid sequence of SEQ lD N°1.

Preferably, in these polypeptides, the amino acid sequence of SEQ ID N°1 is located within the N-terminal end. Most preferably, the N-terminus amino acid residue is selected from the group consisting of (i) the Methionine (also termed M or Met) located at position 1 of SEQ ID N°3, (ii) the Glycine residue (also termed G or Gly) located at position 2 of SEQ ID N°3, the Alanine residue (also termed A or Ala) located at position 3 of SEQ ID N°3, the Arginine (also termed R or Arg) residue located at position 4 of SEQ ID N°3, the Alanine residue located at position 5 of SEQ ID N°3 or the Serine residue (also termed S or Ser) located at position 6 of SEQ ID N°3, the latter amino acid residue consisting also of the N-terminus amino acid residue of SEQ ID N°1.

Illustrative embodiments of polypeptides comprising SEQ ID N°1 encompass proteins comprising the amino acid sequence starting from the Methionine residue located at position 1 of SEQ ID N°3 to the Proline residue located at position 23 of SEQ ID N°3.

In certain preferred embodiments of the Gag-derived polypeptides comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1, the said polypeptides are labelled with a detectable molecule.

According to the invention, the said detectable molecule may consist of any compound or substance that is detectable by spectroscopic, photochemical, biochemical, immunochemical or chemical means.

For example, useful detectable molecules include radioactive substances (including those comprising ³²P, ³⁵S, ³H or ¹²⁵I), fluorescent dyes (including 5-bromodesoxyuridin, fluorescein, acetylaminofluorene or digoxigenin), fluorescent proteins (including GFPs and YFPs), or detectable proteins or peptides (including biotin, polyhistidine tails or other antigen tags like the HA antigen, the FLAG antigen, the c-myc antigen and the DNP antigen).

Most preferably, in the Gag-derived polypeptides comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1 according to the invention, the detectable molecule is located at-, or bound to-, an amino acid residue located outside the said amino acid sequence of interest, in order to minimise or prevent any artefact for the binding of said polypeptides (i) to the candidate compound to be tested or (ii) to the TIP47 protein or to a TIP47-derived polypeptide.

In certain embodiments, a Gag-derived polypeptide comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1 according to the invention consists of a fusion protein with Glutathione S-transferase (GST). In these embodiments, the GST moiety of the said fusion protein may be used as the detectable molecule. In the said fusion protein, the GST moiety may be located either at the N-terminal end or at the C-terminal end. The GST detectable molecule may be detected when it is subsequently brought into contact with an anti-GST antibody, including with a labelled anti-GST antibody. Anti-GST antibodies, including anti-GST antibodies labelled with various detectable molecules are easily commercially available.

In certain other embodiments, a Gag-derived polypeptide comprising an amino acid sequence selected from the group consisting of (i) the amino acid sequence of SEQ lD N°6, (ii) the amino acid sequence of SEQ ID N°7 and (iii) an amino acid sequence having at least 60% amino acid identity with the amino acid of SEQ lD N°1 according to the invention consists of a fusion protein with a poly-histidine peptide. Said poly-histidine peptide usually comprises at least four consecutive histidine residues and generally at least six consecutive histidine residues. Such a poly-histidine peptide may also comprise up to 20 consecutive histidine residues. In theses embodiments, the poly-histidine peptide may be used as the detectable molecule. The poly-histidine detectable peptide may be detected, when it is brought into contact with anti-poly-histidine antibodies, including with labelled anti-poly-histidine antibodies. Anti-poly-histidine antibodies, including labelled anti-poly-histidine antibodies are easily commercially available.

In further embodiments, a Gag-derived polypeptide comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1 according to the invention consists of a fusion protein with a protein moiety consisting of either the DNA binding domain or the activator domain of a transcription factor. The said protein moiety domain of transcription may be located either at the C-terminal end or at the N-terminal end. illustratively, such a DNA binding domain may consist of the well-known DNA binding domain of the LexA protein originating form *E*. *coli*. Further illustratively, such an activator domain may consist of the activator domain of the well-known Gal4 protein originating from yeast.

### Preferred embodiments of the binding partner derived from TIP47.

As already specified above, the MA domain of the HIV-1 Gag protein binds specifically to the cellular TIP47 protein of SEQ ID N°2, and more precisely to a specific protein region of TIP47, starting at the amino acid residue at position 200 and ending at the amino acid residue at position 333 of SEQ ID N°2.

Further, while without wishing to be bound by any particular theory, the applicant believes that the MA domain of the HIV-1 Gag protein binds to a TIP47 peptide region smaller than the 200-333 protein region of SEQ ID N°2 described above.

Thus, for performing step a) of the screening method above using TIP47 peptide fragment as the binding partner, the main, or even the only requirement consists of the use of a binding partner comprising a peptide fragment comprising at least 15 consecutive amino acid residues of the TIP47 protein of SEQ ID N°2, including a peptide fragment comprising at least 15 consecutive amino acid residues of the 200-333 protein region of SEQ ID N°2. Indeed, any one of the peptide fragments of interest has the ability to bind to the HIV-1Gag protein, more precisely to the MA domain of the HIV-1 Gag protein, and even more precisely to the Gag MA domain-derived polypeptides comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1. The said TIP 47 peptide fragments bind to the Gag-derived peptide of SEQ ID N°1. These TIP47 peptide fragments may also be termed herein peptides consisting of TIP47-derived amino acid sequences.

Any one of the TIP47 peptide fragments that are usable for performing a screening method according to the invention binds *in vitro* to the HIV-1 Gag protein, as well as to any polypeptide comprising the Gag-derived amino acid sequence of SEQ ID N°1, preferably with the same order of magnitude than the full length TIP47 protein. This can be easily determined by the one skilled in the art by performing an *in vitro* binding assay, a two-hybrid assay or a HTRF assay, as it is disclosed in the examples herein.

As used herein, a "TIP47-derived" amino acid sequence consists of an amino acid sequence consisting of at least 15 consecutive amino acid residues of the TIP47 protein of SEQ ID N°2. Any one of the TIP47-derived amino acid sequences, whatever its total amino acid length, exclusively consists of a chain of consecutive amino acid residues that are found in SEQ ID N°2.

Such a TIP47-derived peptide fragment may comprise not more than 100 consecutive amino acid residues of the TIP47 of SEQ ID N°2, including not more than 100 consecutive amino acid residues of the 200-333 protein region of the TIP47 of SEQ ID N°2.

In certain embodiments, polypeptides comprising a peptide fragment from TIP47, or from the 200-333 protein region of TIP47, encompass polypeptides comprising at least 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133 or 134 consecutive amino acid residues of SEQ ID N°2.

In these polypeptides, the TIP47-derived amino acid sequence may be located either at the N-terminal end or at the C-terminal end.

In certain preferred embodiments of the polypeptides comprising a TIP47-derived amino acid sequence, said polypeptides are labelled with a detectable molecule, as in certain embodiments of the Gag-derived polypeptides that are previously described above.

According to the invention, the said detectable molecule may consist of any compound or substance that is detectable by spectroscopic, photochemical, biochemical, immunochemical or chemical means.

For example, useful detectable molecules include radioactive substances (including those comprising ³²P, ³⁵S, ³H or ¹²⁵I), fluorescent dyes (including 5-bromodesoxyuridin, fluorescein, acetylaminofluorene or digoxigenin), fluorescent proteins (including GFPs and YFPs), or detectable proteins or peptides (including biotin, polyhistidine tails or other antigen tags like the HA antigen, the FLAG antigen, the c-myc antigen and the DNP antigen).

Most preferably, in the polypeptides comprising a TIP47-derived amino acid sequence according to the invention, the detectable molecule is located at-, or bound to-, an amino acid residue located outside the said TIP47-derived amino acid sequence, in order to minimise or prevent any artefact for the binding of said polypeptides (i) to the candidate compound to be tested or (ii) to the HIV-1 Gag protein or to a peptide fragment thereof comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1.

In certain embodiments, a polypeptide comprising a TIP47-derived amino acid sequence according to the invention consists of a fusion protein with Glutathione S-transferase (GST). In these embodiments, the GST moiety of the said fusion protein may be used as the detectable molecule. The GST detectable molecule may be detected when it is subsequently brought into contact with an anti-GST antibody, including with a labelled anti-GST antibody. Anti-GST antibodies, including anti-GST antibodies labelled with various detectable molecules are easily commercially available.

In certain other embodiments, a polypeptide comprising the TIP47-derived amino acid sequence according to the invention consists of a fusion protein with a poly-histidine peptide. Said poly-histidine peptide usually comprises at least four consecutive histidine residues and generally at least six consecutive histidine residues. Such a poly-histidine peptide may also comprise up to 20 consecutive histidine residues. In theses embodiments, the poly-histidine peptide may be used as the detectable molecule. The poly-histidine detectable peptide may be detected, when it is brought into contact with anti-poly-histidine antibodies, including with labelled anti-poly-histidine antibodies. Anti-poly-histidine antibodies, including labelled anti-poly-histidine antibodies are easily commercially available.

In further embodiments, a polypeptide comprising the comprising the TIP47-derived amino acid sequence according to the invention consists of a fusion protein with a protein moiety consisting of either the DNA binding domain or the activator domain of a transcription factor. illustratively, such a DNA binding domain may consist of the well-known DNA binding domain of the LexA protein originating form *E. coli.* Further illustratively, such an activator domain may consist of the activator domain of the well-known Gal4 protein originating from yeast.

### Preferred embodiments of the screening method using both Gag and TIP47, or peptide fragments thereof.

As it is fully illustrated herein, particularly in the examples herein, the screening for compounds that inhibit the binding between Gag and TIP47 may be performed by detecting an alteration in the binding between the said two binding partners that is induced in the presence of a compound under testing.

Thus in certain embodiments of step a) of the general screening method according to the invention, a method for the screening of compounds that alter the binding between Gag and TIP47, or between suitable peptide fragments thereof, is useful.

Thus in certain embodiments of the method above, step a) consists of a screening method comprising a step of detecting the alteration of the HIV-1 Gag protein/TlP47 protein-protein interactions that is induced by a candidate compound under testing.

Whatever the embodiment of step a) of the screening method above, the complete HIV-1 Gag protein and the complete TIP47 protein may be used as the binding partners.

However, in other embodiments, step a) of the screening method is performed with (1) a polypeptide comprising the Gag-derived amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1 and (2) a polypeptide comprising a TIP47-derived amino acid sequence.

Thus, in certain embodiments of the general *in vitro* screening method of the invention, step a) consists of the following steps :
a1) bringing into contact the candidate compound to be tested with a mixture of
   (1) a first polypeptide comprising an amino acid sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1, and
   (2) a second polypeptide comprising the amino acid sequence of SEQ ID N°2 or a peptide fragment thereof;
a2) determining the ability of said candidate compound to inhibit the binding between said first polypeptide and said second polypeptide.

Various embodiments of both (i) a polypeptide comprising the Gag-derived amino acid sequence and (ii) a polypeptide comprising a TIP47-derived amino acid sequence have previously been described herein, to which it may be referred for performing the screening method above.

In the embodiments of step a) above, what is assayed is the eventual alteration of the binding between (i) the first polypeptide comprising a Gag-derived amino acid sequence and (ii) the second polypeptide comprising a TIP-47 derived amino acid sequence, which binding alteration, when it occurs, is caused by the candidate compound under testing.

Notably, in certain embodiments of the screening method above, said first polypeptide comprises or consists of the polypeptide of SEQ ID N°1, which is optionally labelled with a detectable molecule.

In further embodiments of the screening method above, said second polypeptide comprises or consists of the polypeptide of SEQ ID N° 2 or a peptide fragment thereof, which is optionally labelled with a detectable molecule.

### Preferred embodiments of step a) or a1) of determining the ability of a candidate compound to inhibit the interaction between the HIV-1 Gag protein and TIP47.

Beyond the general methods for measuring the binding between two molecules of interest that have been described previously in the present specification, preferred techniques are described hereunder that may be performed for any one of the embodiments of the general screening method according to the invention, thus whatever the type of step a) or a1) of the said screening method.

However, the techniques hereunder will be described more particularly for performing the embodiment of the screening method according to the invention wherein step a) or a1) involves the binding of
(i) a first polypeptide comprising a Gag-derived amino acid sequence and
(ii) a second polypeptide comprising a TIP47-derived amino acid sequence, in the presence of the candidate compound to be tested.

### Two-hybrid screening assay

In this embodiment of the screening method according to the invention, (i) the first polypeptide comprising a Gag-derived amino acid sequence also comprises a first protein portion of a transcription factor and (ii) the second polypeptide comprising a TIP47-derived amino acid sequence also comprises a second protein portion of a transcription factor, it being understood that the binding together of the first and second protein portions generates a functional transcription factor that binds to a specific regulatory DNA sequence which in turn induces expression of a reporter DNA sequence, said expression being further detected and/or measured. A positive detection of the expression of the said reporter DNA sequence means that an active transcription factor is formed, due to the binding together of (i) the first polypeptide comprising a Gag-derived amino acid sequence and (ii) the second polypeptide comprising a TIP47-derived amino acid sequence.

Usually, in a two-hybrid assay, the first and second protein portions of a transcription factor consist respectively of (i) the DNA binding domain of a transcription factor and (ii) the activator domain of a transcription factor. In some embodiments, the DNA binding domain and the activator domain both originate from the same naturally occurring transcription factor. In some other embodiments, the DNA binding domain and the activator domain originate from distinct naturally occurring transcription factors, while, when bound together, these two protein portions form an active transcription factor. The term "protein portion", when used herein for transcription factor, encompass complete proteins involved in multi-protein transcription factors, as well as specific functional protein domains of a complete transcription factor protein.

Thus, the basic two-hybrid system requires a protein-protein interaction in order to turn on transcription of a reporter gene. Similar systems operating in bacteria and mammalian cells have also been developed and may be used to perform the screening method according to the invention (See Fields et al., Nature 340:245 (1989); Vasavada et al., PNAS USA 88:10686 (1991); Fearon et al., PNAS USA 89:7958 (1992); Dang et al., Mol. Cell. Biol. 11:954 (1991); Chien et al., PNAS USA 88:9578 (1991); and U.S. Pat. Nos. 5,283,173, 5,667,973, 5,468,614, 5,525,490, and 5,637,463).

In some preferred embodiments of a two-hybrid assay for performing the screening method according to the invention, the LexA DNA binding domain and the Gal4 activator domain are used.

In some preferred embodiments of a two-hybrid assay for performing the screening method according to the invention, (i) the first polypeptide comprises the Gag-derived amino acid sequence and the LexA binding domain, and (ii) the second polypeptide comprises the TIP47-derived amino acid sequence and the Gal4 activator domain.

In a preferred embodiment, of the screening method step a2) is performed by measuring the expression of a detectable marker gene placed under the control of a LexA regulation sequence that is responsive to the binding together of the LexA binding domain and the Gal 4 activator domain. For example, the detectable marker gene placed under the control of a LexA regulation sequence may consist of the β-galactosidase gene or the HIS3 gene, as illustratively disclosed in the examples herein.

The general procedure of a specific embodiment of the two-hybrid assay is described hereafter. In an illustrative embodiment, the polynucleotide encoding the first polypeptide comprising the Gag-derived amino acid sequence is fused to a polynucleotide encoding the DNA binding domain of the LexA protein, the fused protein being inserted in a suitable expression vector that is functional in yeast cells.

Then, the polynucleotide encoding the TIP47-derived amino acid sequence is fused to a nucleotide sequence in a second expression vector that also encodes the activation domain of the Gal4 protein.

The two expression plasmids above are transformed into yeast cells and the transformed yeast cells are plated on a selection culture medium which selects for expression of selectable markers on each of the expression vectors as well as GAL4 dependent expression of the HIS3 gene. Transformants capable of growing on medium lacking histidine are screened for gal4 dependent LacZ expression. Those cells which are positive in the histidine selection and the Lac Z assay denote the occurrence of an interaction between the (i) Gag-derived amino acid sequence and (ii) the TIP47-derived amino acid sequence and allow to quantify the binding of together of the two polypeptides, in the presence or absence of the candidate compound to be tested. Further details on the two-hybrid system are described in the examples herein.

Thus, in certain embodiments of the screening method according to the invention, step a) of said method comprises the following steps :
1) providing cells expressing :
   - a first polypeptide consisting of a fusion polypeptide between (i) a Gag-derived amino acid sequence as defined above and (ii) a first protein portion of a transcription factor;
   - a second polypeptide consisting of a fusion polypeptide between (i) a TlP47-derived amino acid sequence as defined above and (i) a second portion of a transcription factor,
   - said transcription factor being active on a DNA target regulatory sequence when the first and second protein portions are bound together, and
   - said cells also containing a nucleic acid comprising (i) a regulatory DNA sequence that may be activated by said active transcription factor and (ii) a DNA reporter sequence that is operably linked to said regulatory sequence;
2) bringing the cells provided at step 1) into contact with a candidate compound to be tested;
3) determining the expression level of said DNA reporter sequence.

At step 1) above, the cells expressing the fusion proteins may be obtained as disclosed in the examples herein. Notably, the one skilled in the art may use the nucleic acid sequences SEQ ID N°4 (HXB2HIV-1 proviral DNA) and SEQ ID N°5 (TIP47) for fusing to the well-known nucleic acids encoding LexA and Gal4.

At step 3), the expression level of said DNA reporter sequence may be assessed by quantifying the amount of the corresponding specific mRNA produced. However, usually, the DNA reporter sequence encodes a detectable protein, so that the expression level of the said DNA reporter sequence is assessed by quantifying the amount of the corresponding protein produced.

Then, the expression level of said DNA reporter sequence that is determined at step 3) above is compared with the expression of said DNA reporter sequence when step 2) is omitted. A lower expression level of said DNA reporter sequence in the presence of the candidate compound mean that the said candidate compound effectively inhibits the binding between HIV-1 Gag and TIP47 and that said candidate compound may be positively selected, at step b) of the screening method.

Detailed protocols for performing two-hybrid assays are provided in the examples herein.

### Western blotting

In another preferred embodiment, of the screening method according to the invention, step a) or a2) comprises a step of subjecting to a gel migration assay the mixture of the first and second polypeptides, with or without the candidate compound to be tested, obtained at the end of step a1) and then measuring the binding of the first polypeptide comprising the Gag-derived amino acid sequence with the second polypeptide comprising the TIP47-derived amino acid sequence by performing a detection of the complexes formed between the said two polypeptides.

The gel migration assay can be carried out as known by the one skilled in the art, for instance as it is shown in the examples.

The detection of the complexes formed between the said two polypeptides may be easily performed by staining the migration gel with a suitable dye and then by determining the stain position (protein bands) corresponding to the proteins analysed since the complexes formed between the first and the second polypeptides possess a specific apparent molecular weight.

In several embodiments, the protein bands corresponding to the proteins submitted to the gel migration assay can be detected by specific antibodies. It may be used both (i) antibodies specifically directed against HIV-1 Gag, or antibodies directed against the MA domain of HIV-1 Gag or also antibodies directed against the HIV-1 Gag MA portion of SEQ ID N°1, and (ii) antibodies directed against TIP47, or antibodies directed against the 200-333 protein portion of TIP47 or also antibodies directed against the suitable peptide fragments of at least 15 consecutive amino acids derived form TIP47 that have been previously described in the present specification.

In several other embodiments, wherein each of the first and second polypeptides are labelled with a detectable antigen, then , the protein bands corresponding to the proteins submitted to the gel migration assay can be detected by specific antibodies directed against said detectable antigen. Preferably, the detectable antigen comprised in the first polypeptide is distinct from the detectable antigen comprised in the second polypeptide. Illustratively, the examples herein disclose embodiments of western blotting assays wherein (i) the first polypeptide comprising a Gag-derived amino acid sequence is labelled by fusion with the GST detectable antigen and (ii) the second polypeptide comprising a TIP47-derived amino acid sequence is labelled by fusion with the HA antigen. For determining and/or quantifying the protein complexes formed between the first and second polypeptides, antibodies directed against the GST antigen and the HA antigen are used, respectively.

Thus, according to these embodiments, step a) of the screening method according to the invention comprises the following steps:
1) providing an assay sample comprising :
   - a first polypeptide comprising a HIV-1 Gag-derived amino acid sequence as defined above;
   - a second polypeptide comprising a TIP47-derived amino acid sequence as defined above; and
   - the candidate compound to be tested;
2) subjecting the assay sample provided at step 1) to migrating on a suitable migration substrate, whereby an assay migration substrate is obtained;
3) detecting and/or quantifying the complexes formed between the first and second polypeptides on the assay migration substrate obtained at the end of step 2).

The presence, or the amount, of the complexes formed between the first and second polypeptides are then compared with the results obtained when at step 1) the assay sample is prepared in the absence of the candidate compound to be tested.

When, at step 3), no complexes between the first and second polypeptides are detected, or alternatively when those complexes are present in a lower amount, compared to the amount of those complexes quantified when step 1) is performed without the candidate to be tested, then the said candidate compound may be positively selected, at step b) of the screening method.

### Biosensor assays

In another preferred embodiment of the screening method above, the screening system used in step (a) includes the use of an optical biosensor such as described by Edwards and Leatherbarrow (Edwards and Leatherbarrow, 1997, Analytical Biochemistry, 246 : 1-6) or also by Szabo et al. (Szabo et al., 1995, Curr. Opinion Struct. Biol., 5(5) : 699-705). This technique allows the detection of interactions between molecule in real time, without the need of labelled molecules. This technique is based on the surface plasmon resonance (SPR) phenomenon. Briefly, a first protein partner molecule, either (i) the first polypeptide comprising a Gag-derived amino acid sequence, or (ii) the second polypeptide comprising the TIP47-derived amino acid sequence, is attached to a surface (such as a carboxymethyl dextran matrix). Then, the second protein partner molecule, either (iii) the second polypeptide comprising the TIP47-derived amino acid sequence or (iv) the first polypeptide comprising a Gag-derived amino acid sequence, is incubated with the previously immobilised first partner, in the presence or in the absence of the candidate compound to be tested. Then, the binding, including the binding level, or the absence of binding between the first and second protein partner molecules is detected. For this purpose, a light beam is directed towards the side of the surface area of the substrate that does not contain the sample to be tested and is reflected by said surface. The SPR phenomenon causes a decrease in the intensity of the reflected light with a specific combination of angle and wavelength. The binding of the first and second protein partner molecules causes a change in the refraction index on the substrate surface, which change is detected as a change in the SPR signal.

According to the preferred embodiment of the screening method cited above, the "first partner" of the screening system consists of the substrate onto which the first protein partner molecule is immobilized, and the "second partner" of the screening system consists of the second partner protein molecule itself.

### Affinity chromatography

Candidate compounds for use in the screening method above can also be selected by any immunoaffinity chromatography technique using any chromatographic substrate onto which (i) the first polypeptide comprising a Gag-derived amino acid sequence, or (ii) the second polypeptide comprising the TIP47-derived amino acid sequence, have previously been immobilized, according to techniques well known from the one skilled in the art.

In a preferred embodiment of the invention, the screening method includes the use of affinity chromatography.

Any one of the first or second polypeptide may be attached to a column using conventional techniques including chemical coupling to a suitable column matrix such as agarose, Affi Gel®, or other matrices familiar to those of skill in the art. In some embodiment of this method, the affinity column contains chimeric proteins in which the first or the second polypeptide, is fused to an antigen labeled, like glutathion -s-transferase (GST). Then a candidate compound is brought into contact with the chromatographic substrate of the affinity column previously, simultaneously or subsequently to the other polypeptide among the said first and second polypeptides. Then, after washing, the chromatography substrate is eluted, and the collected elution liquid is analysed by detection and/or quantification of the said later applied second or first polypeptide, so as to determine if, and/or to which extent, the candidate compound has impaired the binding between (i) the first polypeptide comprising a Gag-derived amino acid sequence and (ii) the second polypeptide comprising the TIP47-derived amino acid sequence.

### Fluorescence assays

In preferred embodiments of the screening method according to the invention, both (i) the first polypeptide comprising a Gag-derived amino acid sequence and (ii) the second polypeptide comprising a TIP47-derived amino acid sequence are labelled with a fluorescent molecule or substance.

According to these preferred embodiments, the eventual alteration effect of the candidate compound to be tested on the binding between the HIV-1 Gag protein and TIP47 is determined by fluorescence quantification.

In certain embodiments, the first and second polypeptides are labelled, for example through protein fusion, with auto-fluorescent proteins, including the well-known GFPs or YFPs proteins.

In certain other embodiments, the first and second polypeptides are labelled with other fluorescent molecules that are suitable for performing fluorescence detection and/or quantification of the binding between the first and the second polypeptide using FRET.

In some embodiments, the first polypeptide and/or the second polypeptide are directly labelled with fluorescent molecules, for example by covalent chemical linkage with the fluorescent molecule. Such embodiments encompass first polypeptide and/or second polypeptide consisting of a fusion protein with an auto-fluorescent molecule like a GFP or a YFP, as well as first and/or second polypeptide having any other fluorescent molecule known in the art which is covalently linked to at least one amino acid residue of said polypeptide.

In some other embodiments, the first and/or the second polypeptide may be indirectly labelled with fluorescent molecules, for example by non-covalent bonding between the said fluorescent molecules and the said polypeptide. Such embodiments encompass first polypeptide and/or second polypeptide comprising a first member of receptor/ligand paired members in its amino acid sequence and the fluorescent molecule comprising the second member of the said receptor/ligand paired members, so that the fluorescent molecule can non-covalently bind to the said first or second polypeptide according to the invention.

An illustrative example of such receptor/ligand paired members consists of the biotin/streptavidin paired members.

Another illustrative example of such receptor/ligand paired members consists of antigen/antibody paired members.

In a preferred embodiment of a first polypeptide comprising a Gag-derived amino acid sequence according to the invention, the said first polypeptide comprises the Gag-derived amino acid sequence that is fused to a detectable antigen, like a poly-histidine tail.

In the embodiment wherein the said detectable antigen consists of GST, then the said first polypeptide may be labelled by non-covalent binding of a fluorescent antigen that is specifically directed against poly-histidine.

In another preferred embodiments of the second polypeptide comprising a TIP47-derived amino acid sequence according to the invention, the said second polypeptide comprises the TIP47-derived amino acid sequence that is fused to a detectable antigen, like the GST antigen.

In the embodiment wherein the said detectable antigen consists of a poly-histidine tail, then the said second polypeptide may be labelled by non covalent binding of a fluorescent antigen that is specifically directed against GST.

Thus, in certain embodiments, the first polypeptide comprising a Gag-derived amino acid sequence is labelled with a first fluorophore substance and the second polypeptide comprising a TlP47-derived amino acid sequence is labelled with a second fluorophore.

As already specified previously, step a) of the screening method according to the invention encompass determination of the ability of the candidate compound to inhibit interaction between the HIV-1 gag protein and TIP47 by fluorescence assays using FRET.

According to this specific embodiment of a fluorescence assay of the invention, either:
- the first fluorophore substance has an emission wavelength value that is substantially equal to the excitation wavelength value of the second fluorophore, whereby the binding of said first and second polypeptides is detected by measuring the fluorescence signal intensity emitted at the emission wavelength value of the second fluorophore substance; or
- the second fluorophore substance has an emission wavelength value that is substantially equal to the excitation wavelength value of the first fluorophore, whereby the binding of said first and second polypeptides is detected by measuring the fluorescence signal intensity emitted at the emission wavelength value of the first fluorophore substance.

The fluorophores used may be of various suitable kinds, such as the well-known lanthanide chelates. These chelates haven been described as having chemical stability, long-lived fluorescence (greater than 0.1 ms lifetime) after bioconjugation and significant energy-transfer in specific bioaffinity assay. U.S. Pat. No. 5,162,508, issued to Lehn, et al. on Nov. 10, 1992 discloses bipyridine cryptates. Polycarboxylate chelators with TEKES type photosensitizers (EP 0203047 A1) and terpyridine type photosensitizers (EP 0649020 A1) are known. International Publication No. WO 96/00901 of Selvin et al., having an International Publication Date of Jan. 11, 1996, discloses diethylenetriaminepentaacetic acid (DTPA) chelates winch used carbostyril as sensitizer. Baley, et al., Analyst, 109, (1984) 1449; Ando, et al. Biochim. Biophys. Acta, 1102, (1992) 186; and Heyduk et al., Anal. Biochemistry, 248, (1997) 216 also describe DTPA lanthanide chelates which contain different sensitizers. Additional DTPA chelates with other sensitizers and other tracer metal are known for diagnostic or imaging use (e.g., EP 0450742 A1).

Most preferably, the fluorescence assay performed at step a) or a2) of the screening methods according to the invention consists of a Homogeneous Time Resolved Fluorescence (HTRF) assay, such as the HTRF assay which disclosed in the PCT application N° WO 00/01663 or in the US patent N° US 6,740,756, the entire content of both documents being herein incorporated by reference.

illustratively, the one skilled in the art can make use of the TRACE technology of fluorescence transfer for Time Resolved Amplified Cryptate Emission for measuring the FRET. This technology allows to set up very specific and very selective and high throughput screening assays for inhibitors of interaction between HIV-1 Gag and TIP47, from which candidate compounds will be selected. This technique is based on the transfer of fluorescence from a donor of energy (cryptate) to an acceptor of energy (XL665), when the two molecules are in close proximity in *in vitro* assays.

The Europium cryptate donor fluorophore has an excitation wavelength value of 337 nm and an emission wavelength value of 620 nm.

The XL665 acceptor fluorophore, which consists of a cross-linked allophycocyanin, has an excitation wavelength value of 620 nm and an emission wavelength value of 665 nm.

Illustratively, at step a) or a1) of the screening methods according to the invention, alteration of the binding between Gag and TIP47 caused by a candidate compound to be tested may be performed as it follows :
1) bringing into contact :
   - a first polypeptide wherein the Gag-derived amino acid sequence is fused to a poly-histidine tail;
   - a second polypeptide wherein the TlP47-derived sequence is fused to GST; and
   - a candidate compound to be tested; during a period of time sufficient for complexes between the first polypeptide and the second polypeptide to be formed, whereby a pre-assay sample is obtained;
2) adding to the said pre-assay sample :
   - anti-poly-histidine antibodies labelled with an Europium cryptate fluorophore; and
   - anti-GST antibodies labelled with a cross-linked allophycocyanin fluorophore (like XL665);
      during a period of time sufficient for the antibodies to bind to their respective target antigens;
      whereby an assay sample is obtained;
3) illuminating the said assay sample obtained at the end of step 2) at the excitation wavelength of the said Europium cryptate fluorophore;
4) detecting and/or quantifying the fluorescence signal emitted at the allophycocyanin compound emission wavelength.

In a further step, the fluorescence signal value which is determined at the end of step 4) above is compared to the signal fluorescence value that is measured when at step 1) the said pre-assay sample is prepared in the absence of the said candidate compound.

If at step 4) above, the intensity value of the fluorescence signal is lower than the intensity value of the fluorescence signal found when step 1) is performed in the absence of the candidate compound under testing, then the said candidate compound may be positively selected, at step b) of the screening method.

Cryptate-labelled anti-GST antibodies encompass those which are commercially available from company CisBio (Bedford, MA, USA), notably those referred to as 61GSTKLA.

Cryptate-labelled anti-poly-histidine antibodies encompass those which are commercially available form company CisBio, notably those referred to as 61HISKLA.

XL665-labelled anti-GST antibodies encompass those which are commercially available from company CisBio, notably those referred to as 61 GSTXLA.

XL665-labelled anti-poly-histidine antibodies encompass those which are commercially available from company CisBio, notably those referred to as 61HISKLB.

Cryptate-labelled or XL665-labelled antibodies directed against other antigens of interest including DNP, c-myc, HA antigen and FLAG antigen are also commercially available from company CisBio.

Illustrative embodiments of a screening method according to the invention using an HTRF assay are disclosed in the examples herein.

### Candidate compounds

According to a first aspect of the screening method above, the candidate compounds, more particularly the candidate inhibitor compounds, may be selected from a library of compounds previously synthesised.

According to a second aspect of the screening method above, the candidate compounds, more particularly the candidate inhibitor compounds, are selected from compounds, the chemical structure of which is defined in a database, for example an electronic database.

According to a third embodiment of the screening method above, the candidate compounds, more particularly the candidate inhibitor compounds, are conceived *de novo.*

The candidate compounds may be selected from the group consisting of (a) proteins or peptides, (b) nucleic acids, and (c) organic or mineral chemical compounds.

Illustratively, libraries of pre-selected candidate nucleic acids may be obtained by the one skilled in the art by performing the SELEX method, using either (i) the first polypeptide comprising a Gag-derived amino acid sequence or (ii) the second polypeptide comprising a TIP47-derived amino acid sequence as the target molecule. For performing the SELEX method, the one skilled in the art may refer to the content of the US patents N° US 5,475,096 and N° US 5,270,163, the content of these two documents being herein incorporated by reference.

Further illustratively, candidate compounds may be selected from the group consisting of (i) antibodies directed against the MA domain of the HIV-1 Gag protein and (ii) antibodies directed against TIP47.

Preferred antibodies encompass the antibodies directed against the Gag-derived peptide of SEQ ID N°1.

Further preferred antibodies encompass the antibodies directed against the 200-333 protein portion of the TIP47 protein of SEQ ID N°2, as well as the antibodies directed to the suitable TIP47 peptide fragments comprising at least 15 consecutive amino acids of the TIP47 of SEQ ID N°2, that are described previously in the present specification.

### Further screening methods according to the invention

The candidate compounds that have been positively selected at the end of any one of the embodiments of the *in vitro* screening method which has been described previously in the present specification may be subjected to further selection steps in view of further assaying its anti-HIV-1 biological properties.

For this purpose, the candidate compounds that have been positively selected with the general *in vitro* screening method above may be further selected for their ability to inhibit the formation of infectious HIV-1 viral particles in HIV-1-infected cells.

Thus, another object of the present invention consists of a method for the *in cellulo* screening of compounds that inhibit formation of HIV-1 virions within cells infected with HIV-1 virus, wherein said method comprises the steps of :
i) screening for compounds that inhibit Env glycoprotein incorporation into HIV-1 virions in cells infected by a HIV-1 virus, by performing the *in vitro* screening method described above; and
ii) screening the compounds positively selected at the end of step i) for their ability to inhibit formation of infectious HIV-1 virions within cells infected with HIV-1 virus.

In certain preferred embodiments of the *in cellulo* screening method above, step ii) of said screening method comprises the following steps :
ii-1) infecting cultured mammalian cells with HIV-1 virus;
ii-2) bringing into contact the infected cells obtained at step ii-1) with a compound that has been positively selected at the end of step i);
ii-3) determining the infectious capacity of the HIV virions produced by the said infected mammalian cells; and
ii-4) comparing the infectious capacity of the HIV virions determined at step ii-3) with the infectious capacity of the HIV virions that is determined when step ii-2) is performed in the absence of the said positively selected compound.

In certain other preferred embodiments of the *in cellulo* screening method above, step ii) of said screening method comprises the following steps :
ii-2) bringing into contact cultured mammalian cells with a compound that has been positively selected at the end of step i);
ii-1) infecting the cultured mammalian obtained at step ii-2) with HIV-1 virus;
ii-3) determining the infectious capacity of the HIV virions produced by the said infected mammalian cells; and
ii-4) comparing the infectious capacity of the HIV virions determined at step ii-3) with the infectious capacity of the HIV virions that is determined when step ii-2) is performed in the absence of the said positively selected compound.

Thus according to the method above, step ii-1) may be performed either prior to, or after, step ii-2), depending of the embodiment under consideration.

Detailed protocol for performing steps ii-1) to step ii-4) are fully provided in the examples herein.

For performing step ii-1), mammalian cells encompass primary culture cells as well as cell lines. Primary culture cells include primary cultures from mammalian peripheral blood mononuclear cells (PBMC), primary cultures from mammalian peripheral blood lymphocytes and primary cultures from mammalian monocytes or mammalian macrophages. In some embodiments, primary culture cells are preactivated before their use at step ii-1) of the method above. Mammalian cells also encompass mammalian cells originating from various mammalian cell lines, preferably mammalian cell lines expressing CD4 receptors at their membrane surface. Preferred mammalian cells consist of human cells.

Illustratively, step ii-1) may be performed by infecting HeLa cells expressing CD4 receptors, for example by cell transfection with the well-known HIV-1 HXB2R molecular clone.

Illustratively, step ii-2) may be performed by adding an amount of the candidate compound to be tested to the culture medium wherein the (i) already HIV-infected, or (ii) not yet HIV-infected, HeLa cells are cultured. Usually, a plurality of culture samples are prepared, so as to add increasing amounts of the candidate compound to be tested in distinct culture samples. Generally, at least one culture sample without candidate compound is also prepared as a negative control, for further comparison. Optionally, at least one culture sample with an already known inhibitor compound is also prepared as a positive control, for standardisation of the method. Such known inhibitor compound may consist of a siRNA specific for TlP47.

Illustratively, determining the infectious capacity of the HIV virions at step ii-3) may be performed by quantification of the p24 Gag antigen in the cell culture supernatants of the cells infected at step ii-1), including by performing an ELISA assay with anti-p24 antibodies. Preferably, in these embodiments, p24 Gag antigen is quantified only after at least a 3 days culture time period following HIV-1 infection, so as to easily discriminate between production of infectious and non-infectious HIV-1 virions. Usually, optimal p24 Gag antigen quantification is performed after a 5 days culture time period following HIV-1 infection.

Further illustratively, determining the infectious capacity of the HIV virions at step ii-3) may be performed by determining the infectious capacity of the HIV-1 virus particles present in the culture supernatants of the infected mammalian cells, using indicator cells. The said indicator cells are distinct from the cells infected at step ii-1). In these embodiments of step ii-3), cell culture supernatants from the cells infected at step ii-1) are collected and then brought into contact with cultured indicator cells which undergo detectable physiological changes upon infection with infectious HIV-1 virions. Then, detection and/or quantification of the said physiological changes in the cultured indicator cells allow determination of the infectious capacity of the virions particles that were produced by the cells infected at step ii-1). Preferably, in these embodiments, cell culture supernatants from the cells infected at step ii-1) are collected after a time period ranging from 6 hours to 48 hours, most preferably about 24 hours, following HIV-1 infection. Preferably, in these embodiments, detection of physiological changes within the cultured indicator cells is performed after a time period ranging from 24 hours to 120 hours (5 days), preferably from 24 hours to 48 hours, following the addition of the cell culture supernatants of the cells infected at step ii-1). Illustratively, the said indicator cells encompass HeLa P4-2 cell line which expresses CD4 and contains an integrated *lacZ* reporter gene driven by the HIV LTR, these indicator cells becoming blue upon infection with infectious HIV-1 virions.

Illustratively, in the embodiments wherein the infectious capacity of the HIV-1 virions is determined according to the amount pf p24 GAG, step ii-4) may be performed by comparing the p24 Gag ELISA quantification data obtained for the cell cultures incubated with the candidate compound to be tested with the p24 Gag ELISA quantification data obtained for the negative control cell cultures without the said candidate compound nor the known inhibitor compound.

A lower amount of p24 Gag antigen in the cell cultures incubated with the said candidate compound mean that the said candidate compound effectively inhibits the formation of infectious HIV-1 viral particles.

Further, the efficiency of the inhibitor candidate compound may be assessed by comparing (i) the amount of p24 Gag antigen measured in the supernatant of the cell cultures that were incubated therewith with (ii) the amount of p24 Gag antigen measured in the supernatant of the positive control cell cultures incubated with the known inhibitor, for instance the positive control cell cultures incubated with the siRNA specific for TIP47.

Still further, the efficiency of the inhibitor candidate compound may be assessed by determining for which amount of the candidate compound added to the cell cultures the amount of p24 Gag protein is close to the amount of p24 Gag protein found for the known inhibitor, including the known siRNA specific for TIP47.

In the embodiments wherein the infectious capacity of the HIV-1 virions is determined according to induction of physiological changes in cultured indicator cells, step ii-4) may be performed by comparing the color change quantification data obtained for the cell cultures incubated with the candidate compound to be tested with the color change quantification data obtained for the negative control cell cultures without the said candidate compound nor the known inhibitor compound.

As intended herein, quantification of the level of color change in the cultured indicator cells encompass quantification by counting the number of colored cells in each cell culture sample, e.g. the number of blue cells in each culture sample. Thus, the level of color change increases with increasing number of colored (e.g. blue) cells in the cell culture samples, whereas the level of color change decreases with decreasing number of colored (e.g. blue) cells in the cell culture samples.

A lower color change in the indicator cells, after contact with the culture supernatant from the cell cultures infected with HIV-1 and incubated with the said candidate compound mean that the said candidate compound effectively inhibits the formation of infectious HIV-1 viral particles.

Further, the efficiency of the inhibitor candidate compound may be assessed by comparing (i) the level of color change in cultured indicator cells incubated with the supernatant of the cell cultures infected with HIV-1 and that were incubated with the said inhibitor candidate with (ii) the level of color change in cultured indicator cells incubated with the supernatant of the positive control cell cultures incubated with the known inhibitor, for instance the positive control cell cultures incubated with the siRNA specific for TIP47.

Still further, the efficiency of the inhibitor candidate compound may be assessed by determining for which amount of the candidate compound added to the cell cultures infected with HIV-1, the level of color change in cultured indicator cells is close to the level of color change in cultured indicator cells found for the known inhibitor, including the known siRNA specific for TIP47.

### Inhibitors of the production of infectious HIV-1 viral particles

The present invention also pertains to inhibitors of the production of infectious HIV-1 viral particles, that possess the ability of inhibiting or preventing the binding between HIV-1 Gag and TIP47 in mammalian cells, specifically in human cells, infected with a HIV-1 virus.

These inhibitors compounds, due to their property of inhibiting the binding between HIV-1 Gag and TIP47 in HIV-1-infected cells, cause the production of defective HIV-1 viral particles by the said infected cells, namely HIV-1 viral particles having a reduce content in gp41 and gp120 glycoproteins. Those defective HIV-1 viral particles have at least reduced infectious properties, due to the lack of gp120-mediated binding of the defective viral particles with target cells.

The impairment, by any one of the inhibitor compounds that may be positively selected through the screening method according to the present invention, of the release of infectious viruses by HIV-1-infected cells will prevent the spreading of HIV-1 in infected individuals. Thus, an inhibitor compound according to the invention should cause a reduction in the HIV-1 viral load in infected individuals and improve efficiency of the anti-HIV-1 immune response, as well as the efficiency of combined therapy.

In certain embodiments of these inhibitor compounds, those inhibitor compounds consist of compounds that mimic the HIV-1 Gag protein that is naturally produced in HIV-1-infected cells, although these inhibitor compounds lack the ability of the natural Gag protein to participate in the HIV-1 particles formation. Such inhibitor compounds act as lures for the infected cell machinery by sequestering the cellular TIP47 protein, which TlP47 protein is then no more available for binding with the HIV-1 Gag protein expressed by the HIV-1 mRNA.

In certain embodiments of these inhibitor compounds, those inhibitor compounds consist of compounds that mimic TIP47, although these inhibitor compounds lack the ability to connect the Gag and Env glycoproteins that are produced in HIV-1-infected cells. Such inhibitor compounds act as lures for the infected cell machinery by sequestering the Gag protein expressed by the HIV-1 mRNA, which Gag protein is no more available for binding with the TIP47 protein expressed by the cellular mRNA.

Thus, in several preferred embodiments of the inhibitor compounds above, said inhibitor compounds consist of any one of the Gag-derived peptides that are disclosed in the present specification.

Thus, inhibitor compounds according to the invention encompass peptides comprising a Gag-derived peptide sequence selected from the group consisting of :
(A) the peptide consisting the amino acid sequence of SEQ ID N° 6 below :
   NH₂-S-X₄-L-X₅-X₆-X₇-X₈-X₉-X₁₀-X₁₁-W-E- COOH, wherein
      - X4 is an amino acid residue selected from the group consisting of V, L, I, T, R and E;
      - X5 is an amino acid residue selected from the group consisting of S, T, R, I and K;
      - X6 is an amino acid residue selected from the group consisting of G and A;
      - X7 is an amino acid residue selected from the group consisting of G, R, K, E, S, T, K and D;
      - X8 is an amino acid residue selected from the group consisting of E, K, R, Q and G;
      - X9 is an amino acid residue selected from the group consisting of L, F, K and Q;
      - X10 is an amino acid residue selected from the group consisting of D and E; and
      - X11 is an amino acid residue selected from the group consisting of R,A,S,K,Q,T,E,N,D
(B) the peptide consisting the amino acid sequence of SEQ ID N° 7 below :
   NH₂-X₁-X₂-A-X₃-A-S-X₄-L-X₅-X₆-X₇-X₈-X₉-X₁₀-X₁₁-W-E-X₁₂-X₁₃-X₁₄-X₁₅-X₁₆-P-COOH, wherein
      - X1 is an amino acid residue selected from the group consisting of M, L, V and I;
      - X2 is an amino acid residue selected from the group consisting of G, S and T;
      - X3 is an amino acid residue selected from the group consisting of R, G, S and K;
      - X4 is an amino acid residue selected from the group consisting of V, L, I, T, R and E;
      - X5 is an amino acid residue selected from the group consisting of S, T, R, I and K;
      - X6 is an amino acid residue selected from the group consisting of G and A;
      - X7 is an amino acid residue selected from the group consisting of G, R, K, E, S, T, K and D;
      - X8 is an amino acid residue selected from the group consisting of E, K, R, Q and G;
      - X9 is an amino acid residue selected from the group consisting of L, F, K and Q;
      - X10 is an amino acid residue selected from the group consisting of D and E;
      - X11 is selected from the group consisting of R, A, S, K, Q, T, E, N, D and P;
      - X12 is an amino acid residue selected from the group consisting of K, R, N, E, A and S;
      - X13 is an amino acid residue selected from the group consisting of I, M, Q and T;
      - X14 is an amino acid residue selected from the group consisting of R, K, W, Q, H and Y;
      - X15 is an amino acid residue selected from the group consisting of L and S; and
      - X16 is an amino acid residue selected from the group consisting of R and K.
   and
(C) a peptide ahving at least 60% amino acid identity with the Gag amino acid sequence of SEQ lD N°1.

In one embodiment, said inhibitor compounds consist of peptides comprising the Gag-derived amino acid sequence of SEQ ID N°1.

In a further embodiment, said inhibitor compounds consist of peptides having not more than 100 amino acid residues in length and comprising the amino acid sequence of SEQ ID N°1.

In a still further embodiment, said inhibitor compounds consist of peptides comprising not more than 100 consecutive amino acid residues of the Gag protein of SEQ ID N°3 and comprising the amino acid sequence of SEQ ID N°1. These peptides encompass those comprising 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 consecutive amino acid residues of SEQ ID N°3 and comprising the amino acid sequence of SEQ ID N°1.

Thus, in several preferred embodiments of the inhibitor compounds above, said inhibitor compounds consist of any one of the TIP47-derived peptides that are disclosed in the present specification.

More precisely, such inhibitor compounds encompass those peptides having at least 15 consecutive amino acid residues of the amino acid sequence starting at position 200 and ending at position 333 of the TIP47 amino acid sequence of SEQ ID N°2.

Thus, in preferred embodiments, such inhibitor compounds consist of peptides comprising, or consisting of, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133 or 134 consecutive amino acid residues of SEQ ID N°2, those consecutive amino acid residues being preferably comprised in the amino acid sequence starting at position 200 and ending at position 333 of the TIP47 amino acid sequence of SEQ ID N°2.

The present invention also relates to compositions comprising at least one inhibitor compound as defined above in combination with one or more auxiliary substances.

Any one of the inhibitor compounds that are described herein may be used as an active ingredient for manufacturing pharmaceutical compositions for preventing or treating a disease linked to the infection of an individual with a HIV virus, specifically with a HIV-1 virus.

### Pharmaceutical compositions and methods.

The present invention also pertains to pharmaceutical compositions comprising at least one HIV-1 inhibitor compound as described above, in combination with one or more physiologically acceptable excipients.

The present invention also concerns prophylactic and therapeutic methods comprising a step of administering an inhibitor compound or a pharmaceutical composition as defined above, to an individual in need thereof.

The individuals in need of such treatments encompass those which are susceptible to be in contact with HIV-1 viruses, due to their personal or professional activities, or also their social behaviour.

By "physiologically acceptable excipient or carrier" is meant solid or liquid filler, diluent or substance which may be safely used in systemic or topical administration. Depending on the particular route of administration, a variety of pharmaceutically acceptable carriers well known in the art include solid or liquid fillers, diluents, hydrotropes, surface active agents, and encapsulating substances.

Pharmaceutically acceptable carriers for systemic administration that may be incorporated in the composition of the invention include sugar, starches, cellulose, vegetable oils, buffers, polyols and alginic acid. Specific pharmaceutically acceptable carriers are described in the following documents, all incorporated herein by reference: U.S. Pat. No. 4,401,663, Buckwalter et al. issued August 30, 1983; European Patent Application No. 089710, LaHann et al. published Sept. 28, 1983; and European Patent Application No. 0068592, Buckwalter et al. published Jan. 5, 1983. Preferred carriers for parenteral administration include propylene glycol, pyrrolidone, ethyl oleate, aqueous ethanol, and combinations thereof.

Representative carriers include acacia, agar, alginates, hydroxyalkylcellulose, hydroxypropyl methylcellulose, carboxymethylcellulose, carboxymethylcellulose sodium, carrageenan, powdered cellulose, guar gum, cholesterol, gelatin, gum agar, gum arabic, gum karaya, gum ghatti, locust bean gum, octoxynol 9, oleyl alcohol, pectin, poly(acrylic acid) and its homologs, polyethylene glycol, polyvinyl alcohol, polyacrylamide, sodium lauryl sulfate, poly(ethylene oxide), polyvinylpyrrolidone, glycol monostearate, propylene glycol monostearate, xanthan gum, tragacanth, sorbitan esters, stearyl alcohol, starch and its modifications. Suitable ranges vary from about 0.5% to about 1%.

For formulating a pharmaceutical composition according to the invention, the one skilled in the art will advantageously refer to the last edition of the European pharmacopoeia or of the United States pharmacopoeia.

Preferably, the one skilled in the art will refer to the fifth edition "2005" of the European Pharmacopoeia, or also to the edition USP 28-NF23 of the United States Pharmacopoeia.

The weight amount of therapeutically active compound that is contained in each dose of the pharmaceutical composition of the invention will depend on the molecular weight of said therapeutically active compound as well as on the weight amount that is effective in inhibiting or blocking the fully infectious HIV-1 production in individuals infected with an HIV virus.

For determining the appropriate amount of the therapeutically active compound, in a dose of a pharmaceutical composition of the invention, the one skilled in the art firstly determines the HIV1 replication and virion production ability of various weight amounts or concentrations of said therapeutically active compound, for example by performing the same steps as in the screening method of the invention which has been previously described herein, and then retain or select the given amount or concentration of said therapeutically active compound that inhibits or blocks the fully infectious HIV-1 production in individuals infected with an HIV virus. Then, the one skilled in the art adapts said retained or selected amount or concentration to the *in vivo* human situation, so that the concentration of said therapeutically active compound in the blood of a patient to which the pharmaceutical composition of the invention has been administered is identical to the concentration that significantly reduces or blocks the fully infectious HIV-1 production *in vitro.*

The one or more inhibitors compounds according to the invention, when used for manufacturing pharmaceutical compositions, or when used for administration to individuals in need thereof, may be combined with one or more other anti-HIV-1 active ingredients like, for example, one or more reverse transcriptase inhibitors, and/or one or more protease inhibitors.

The present invention is further illustrated by the examples below.

### EXAMPLES

### A. MATERIALS AND METHODS

### A.1. Two-hybrid assay.

We generated FBL-p18-24, FBL-CAp24, FBL-MAp18 and FBL-MA₁₋₂₃ constructs by fusing DNA fragments encoding respectively amino acid residues 1 to 362, 133 to 362, 1 to 132 and 1 to 23 from the Gag open reading frame (ORF) at the N-terminus of the LexA binding domain.

The pGAD-TlP47 plasmid encoding the full-length ORF of *TlP47* fused to the Gal4 activation domain (Gal4 AD) has been previously described ( Blot, G., Janvier, K., Le Panse, S., Benarous, R., and Berlioz-Torrent, C. (2003). Targeting of the human immunodeficiency virus type 1 envelope to the trans-Golgi network through binding to TIP47 is required for env incorporation into virions and infectivity. J Virol 77, 6931-6945).

Mutated FBL-MA₁₋₂₃ vectors were generated by PCR-directed mutagenesis with the appropriate primers. Liquid culture assays for quantitative β-gal activity and interaction assays were carried out in yeast L40 reporter strains containing the *HIS3* and β̃ GalLexA-inducible genes, as previously described (Blot *et al.,* 2003, *Supra).* Yeast proteins were extracted in lysis buffer (0.04 M KCI, 5 mM Tris pH 7.9, protease inhibitor cocktail (Sigma)) in presence of glass beads. The extracted proteins were then separated by SDS-PAGE and subjected to western blotting with anti-LexA Ab (Invitrogen).

### A.2. Cell culture and plasmids.

HeLa, 293T and HeLa P4-2 cells (Clavel and Charneau, 1994) were grown in DMEM, and Jurkat cells were grown in RPMI 1640. These media were supplemented with glutamine, antibiotics and 10 % FCS. HeLa P4-2 cells were supplemented with 100µg of geneticin/ml.

### A.3. TIP47 binding assays.

GST-MAp18, GST-CAp24 and GST-TIP47 were obtained from direct insertion of respectively matrix, capsid and TIP47 ORFs into pGex4T-1 vector.

HeLa cells were transfected by electroporation with 10µg pAS1B-HA-TIP47 (Blot *et al.,* 2003, *Supra*)*.* After 48h, HA-TIP47-transfected cells (5x10⁶) were lysed, (50 mM Tris, 150 mM NaCl, 5 mM EDTA, 1% Triton X-100, pH8) and incubated with 5 µg of purified GST or GST-ENV HIV, GST-MAp18, GST-CAp24 or GST-TIP47 proteins on gluthatione-sepharose beads.

The beads were washed and bound cellular proteins were separated by SDS-PAGE and subjected to western blotting with anti-HA mAb (3F10, Roche) as previously described ( Berlioz-Torrent, C., Shacklett, B. L., Erdtmann, L., Delamarre, L., Bouchaert, I., Sonigo, P., Dokhelar, M. C., and Benarous, R. (1999). Interactions of the cytoplasmic domains of human and simian retroviral transmembrane proteins with components of the clathrin adaptor complexes modulate intracellular and cell surface expression of envelope glycoproteins. J Virol 73, 1350-1361).

### A.4. Co-immunoprecipitation experiments.

We generated MA S₅V₆-AA and MA W₁₅E₁₆-AA mutant HXB2R proviral clones by site-directed mutagenesis on the *Nar*I*-Spe*I fragment (nt 655 to 1524) from HXB2R proviral DNA using the appropriate primers.

We verified the mutations by sequencing and insertion into HXB2R. We transiently transfected, using the Lipofectamine Plus reagent (Invitrogen), 2x10⁵ HeLa cells with either 1µg of pAS1B-HA-TIP47 or pAS1B vectors, and 1µg of one of the following: HXB2R wt (wild type), HXB2R ΔENV (a virus that cannot express Env products; from F. Mammano), pMA243 (a virus that cannot express Gag products; from M. Alizon), HXB2R Env Y₈₀₂W₈₀₃-SL (Blot *et al.,* 2003) (a virus with mutated Y₈₀₂W₈₀₃ Env residues), HXB2R MA S₅V₆-AA or HXB2R MA W₁₅E₁₆-AA proviral DNA (virus with mutated S₅V₆ or W₁₅E₁₆ MA residues).

Cells were harvested 48 h after transfection and lysed in lysis buffer (1 % Triton X-100, 150 mM NaCl, 10 mM Tris HCl (pH 7.5), 0.1% DOC, 1 mM EDTA, and protease inhibitor cocktail).

For immunoprecipitation, the cell lysates were incubated overnight with protein G-Sepharose beads previously incubated with anti-HA (12CA5, Roche) or anti-TMgp41 (41A, Hybridolabs) antibodies, and then washed six times with the lysis buffer. Bound cellular proteins were separated by SDS-PAGE and subjected to western blotting using rabbit anti-CAp24 (NIH), human anti-TMgp41 (2F5, NIH) and rat anti-HA (3F10) antibodies.

For co-immunoprecipitation with siRNA against TlP47, 2x10⁵ HeLa P4-2 cells were transfected with 30nM of either TIP-A or control Luc siRNA and either pcDNA3 or HXB2R wt proviral DNA using the Lipofectamine Plus reagent. After 24h, the cells were transfected again with 30nM siRNA using Oligofectamine reagent (Invitrogen). After 48h, the cells were lysed and immunoprecipitated with anti-TMgp41 antibodies as described above.

### A.5. Viral replication kinetics.

Viral stocks of HIV-1 HXB2R wt, MA S₅V₆-AA, MA W₁₅E₁₆-AA or Env Y₈₀₂W₈₀₃-SL mutants were obtained by transfecting 293T cells (2x10⁶) with 20µg of the corresponding HXB2R proviral DNA using the calcium phosphate procedure (Stratagene). After 48h, the supernatants of the transfected cells were collected, filtered through a 0.45µm pore filter, quantified for HIV-1 p24 antigen using a Coulter HIV-1 p24 antigen assay (Beckman Coulter), and used in the infection assay on Jurkat T cells, as described previously (Blot *et al.,* 2003, *Supra).*

### A.6. Multiple rounds of infection.

CD4⁺ HeLa P4-2 cells (4x10⁵ cells) were transfected twice, 24 hours apart, with 30 nM siRNA (TIP-A sense (positions 15-33): 5'tgaagtcgcggccgctgtt3'; TIP-B sense (positions 495-512): 5'ggacacgguggccacccaatt3'; Luc sense (positions 153-173), 5'cguacgcggaauacuucga3') using Oligofectamine reagent. HXB2R HIV-1 virus-containing supernatants (30 ng of p24, M.O.I.: 0.05) were used to infect HeLa P4-2 cells 24 h after the second transfection.

The following day, the cells were washed in PBS and overlaid with fresh growth medium. The cells and supernatants were harvested 96 h later and p24 Gag antigen production in the supernatants was quantified by ELISA. We checked for TIP47 expression by western blotting using rabbit anti-TIP47 antibodies.

### A.7. HIV-1 entry assay.

CD4⁺ HeLa P4-2 cells (2x10⁵ cells) were transfected twice, 24 hours apart, with 30 nM siRNA using Oligofectamine reagent. HXB2R HIV-1 virus-containing supernatants (100 ng of p24) were used to infect HeLa P4-2 indicator cells 24h after the second transfection. The cells were washed 12h later and overlaid with fresh growth medium containing the CXCR4 inhibitor AMD3100 (100ng/ml). After 24h, the cells were washed in PBS, fixed with 0.5% glutaraldehyde and assayed for β-gal activity. The number of infected cells was determined by counting the number of blue cells indicating β-gal activity.

### A.8. HIV-1 production assay.

HeLa cells (4x10⁵ cells) were transfected with the HIV-1 HXB2R molecular clone and 30 nM siRNA using Lipofectamine Plus reagent. After 24h, the cells were washed and transfected with 30 nM of siRNA using Oligofectamine reagent. The cells and supernatants were analysed 48h after transfection.

Cell lysates were separated by SDS-PAGE and proteins were subjected to western blotting with anti-p24 (ARP366), rabbit anti-TIP47 and anti-tubulin (DM1A, Sigma) antibodies. We quantified p24 Gag antigen production in the supernatants by ELISA and used the supernatants to infect CD4⁺ HeLa P4-2 indicator cells to determine the titer of the produced virus.

### A.9. Incorporation assays.

Incorporation assays in Jurkat T cells were carried out as previously described (Blot *et al.,* 2003, *Supra*)*.* For incorporation assays in HeLa cells, we transfected 5x10⁶ HeLa cells with 4 µg of HIV-1 HXB2R wt, MA W₁₅E₁₆-AA or Env Y₈₀₂W₈₀₃-SL proviral DNA using Lipofectamine 2000 reagent.

For incorporation assays in presence of overexpressed TIP47, cells were transfected with 4 µg HIV-1 HXB2R wt proviral DNA and 4µg of either pAS1B-HA-TIP47 or pAS1B vectors.

For incorporation assays in the presence of siRNA, HeLa (4x10⁵ cells) were transfected twice, 24 hours apart, with 1µg HIV-1 HXB2R proviral DNA, 30 nM siRNA and 0.5µg of either pASIB or pHA-TIP47 using Lipofectamine Plus reagent.

After 24h, cells were washed and cultured for two days in DMEM medium. The supernatants were then collected, filtered through a 0.45 µm pore filter and quantified for HIV-1 p24 antigen using a Coulter HIV-1 p24 antigen assay.

They were then used in infection assays and ultracentrifuged at 27,000 rpm for 1 h20 in the rotor Surespin 630 (Sorvall). The pellets were resuspended in 80 µl of Laemmli buffer. Cell and virion lysates were separated by SDS-PAGE and subjected to western blotting with anti-TMgp41 (2F5), rabbit anti-p24 (NIH), rabbit anti-TIP47 and anti-HA (3F10) antibodies.

### A.10. HTRF Assay

### A.10.1. Cloning procedures:

pGST-TIP47 construct was obtained from direct insertion of TIP47 ORF into pGex4T-1 vector (Blot G, Janvier K, Le Panse S, Benarous R, Berlioz-Torrent C. Targeting of the human immunodeficiency virus type 1 envelope to the trans-Golgi network through binding to TIP47 is required for env incorporation into virions and infectivity. J Virol. 2003 Jun; 77(12):6931-45). The Matrix domain (MA) of Gag (amino acid 1 to 132 of Gag ORF) was cloned into pHGWA as described by Busso *et al* ( Busso D, Delagoutte-Busso B, Moras D Construction of a set Gateway-based destination vectors for high-throughput cloning and expression screening in Escherichia coli. Anal Biochem. 2005 Aug 15;343(2):313-21). The resulting vector obtained is pHGWA-MA-His6. This vector encodes the MA domain fused to a C-terminal six-histidine (His6) tag, named MA-His6.

### A.10.2. Protein purification procedures:

pGST-TIP47 and pHGWA-MA-His6 were transformed, respectively, in competent DH5α and *E*. *coli* BL21 (DE3) cells. Transformed cells were used to inoculate a starter culture (10ml) in LB-ampicillin that grown for 18 h at 37°C to reach saturation. The starter culture were then used to inoculate a culture of LB-ampicillin (800 ml) at OD₆₀₀ₙₘ=0.1. After 2-3h at 37°C in a rotating shaker (OD₆₀₀ₙₘ=1-2), IPTG was added (Final concentration: 1mM) and the cultures were grown for an additional 5h. Cells were harvested by centrifugation and the cell pellets were washed with NaCl 1%. The pellets were lysed, sonicated into buffer A (NaCl 300 mM, Hepes pH 7.0 40 mM, MgCl2 5 mM, glycérol 5 %, CHAPS 7 mM, β-mercaptoethanol 2 mM, protease inhibitors) and ultracentrifugated. The supernatants were loaded on affinity resin (Glutathion Sepharose 4B beads for GST TIP47 or Talon BD superflow metal affinity resin for MA-His6x). After 1 h incubation at 4°C, beads were washed two times with buffer A. GST-TIP47 protein was eluted with buffer A in presence of 20 mM Glutathion. MA-His6x was eluted with a gradient of imidazole (from 25 mM to 200 mM) in buffer A. The purified proteins were quantified by Bradford and analysed on SDS/PAGE gel by Commassie blue.

### A.10.3. HTRF (Homogeneous Time-Resolved Fluorescence):

HTRF technology is based on fluorescence resonance energy transfer (FRET). In HTRF technology, Eu3+ cryptate molecule coupled to Anti-His antibodies and XL665 molecules coupled to anti-GST antibodies are, respectively, the donor and the acceptor. When, the two entities come into close proximity (for example, in presence of an interaction between two partners) and upon excitation at 337 nm, FRET occurs and XL665 re-emits a specific long-lived fluorescence at 665 nm. Same quantities of purified proteins, (in the range of 50 ng to 500 ng) of GST-TIP47 and MA-His6x, were incubated into 20µl of HTRF Buffer (50 mM phosphate buffer pH 7.0, 0.8 M Kalium Fluoride) in presence of 2.9 ng of Anti-His-Cryptate (Cisbio) and 20 ng of Anti-GST-XL665 (Cisbio). The specific long-lived fluorescence at 665 nm was quantified with Rubystar Reader (BMG).

### EXAMPLE 1 : Requirement of TIP47 for full infectivity of HIV-1.

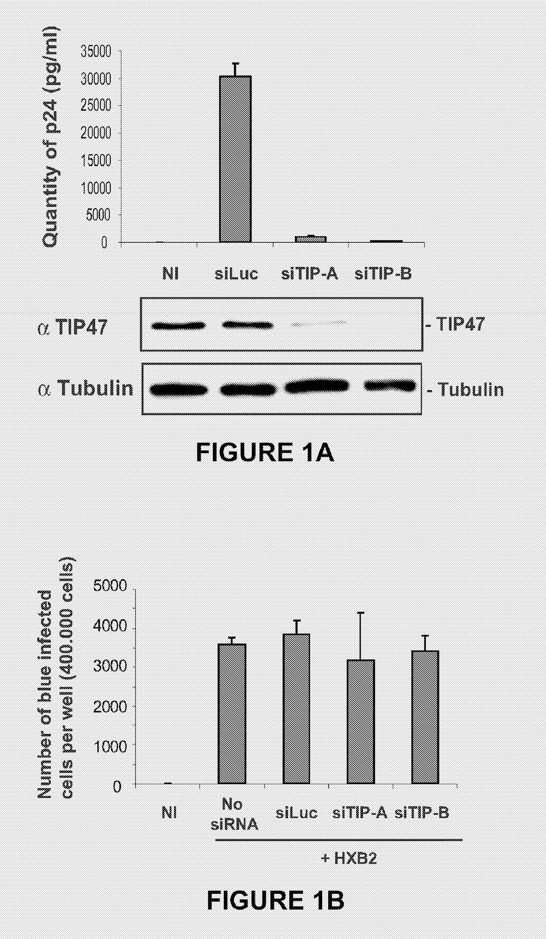

We investigated the role of cellular TIP47 in HIV-1 replication by infecting CD4⁺ HeLa P4-2 cells previously depleted of TIP47 by siRNA treatment with HIV-1 HXB2R. After multiple rounds of infection during 96h, the silencing of TIP47 strongly inhibited HIV-1 replication (greater than 30 times less replication with siTIP-A and siTIP-B than with siLuc, Fig. 1A), whereas we observed no effect after treatment with siRNA against the luciferase GL2 sequence ( Elbashir, S. M., Harborth, J., Lendeckel, W., Yalcin, A., Weber, K., and Tuschl, T. (2001). Duplexes of 21-nucleotide RNAs mediate RNA interference in cultured mammalian cells. Nature 411, 494-498.) used as a control (denoted Luc siRNA). We observed HXB2R replication inhibition with two different siRNAs, siTIP-A and siTIP-B, which targeted the 5' untranslated region and the coding sequence of the TIP47 mRNA, respectively. These results suggest that TIP47 is essential for HIV-1 spreading.

The inhibition of viral propagation that we observed in Hela P4-2 upon the silencing of TIP47 may be due to a defect in either the early steps of infection or the later steps of production of infectious viruses. We tested the first hypothesis by infecting HIV-1 LTR-LacZ HeLa P4-2 indicator cells depleted of TIP47 by siRNA treatment with HIV-1 HXB2R. After infection, the cells were maintained in the presence of AMD31 00, a CXCR4 inhibitor, to avoid multiple rounds of reinfection. In these conditions, β-galactosidase production by these cells, resulting from Tat expression during a single round infectivity assay, was not affected by TIP47 silencing.

These results show that the involvement of TIP47 in HIV-1 replication does not occur in the first steps of the viral cycle (Fig. 1B).

### EXAMPLE 2 : Depletion of TIP47 in producer cells abolishes Env incorporation into virions

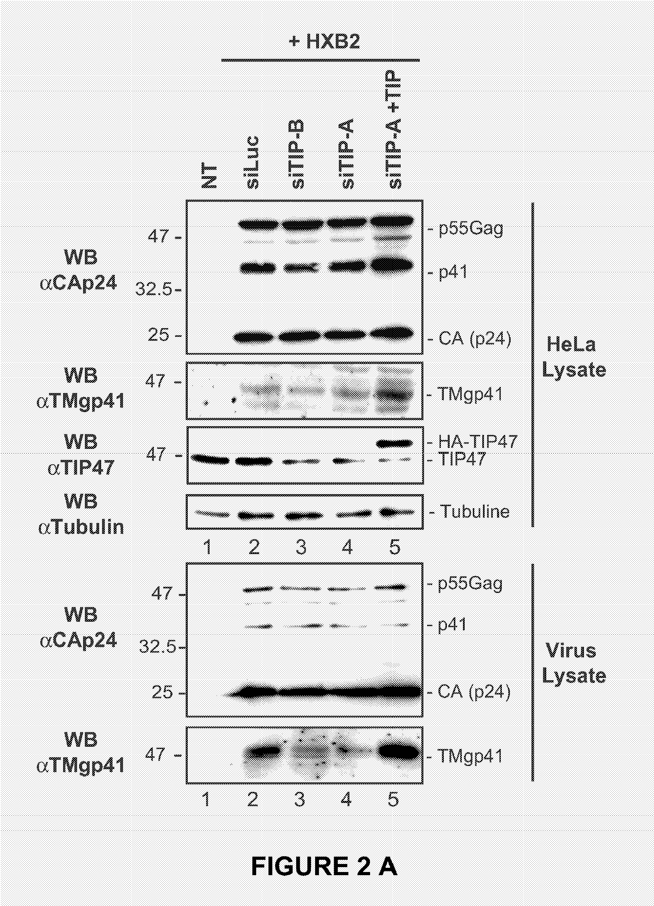

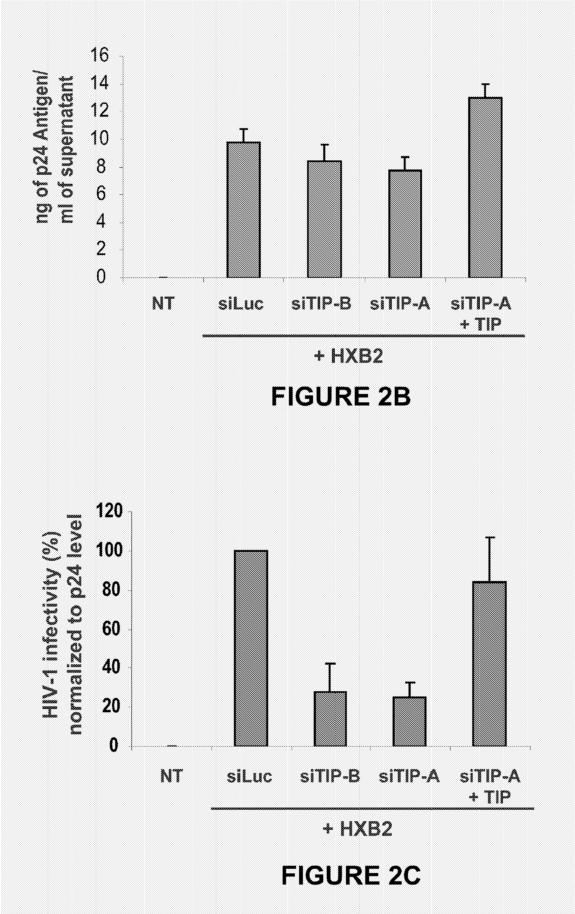

We next analysed the effect of TIP47 depletion on the later steps of production of infectious viruses. We transfected TIP47-depleted HeLa cells with HIV-1 HXB2R provirus DNA to obtain viral stocks. Western blots of producer cell lysates using CAp24 and TMgp41 antibodies showed similar intracellular levels of HIV-1 TMgp41 subunit and Gag products (Pr55 Gag, p41 and CAp24) in cells treated with siRNAs against TIP (siTIP-A or siTIP-B) or with siRNA Luc (Fig 2A, upper panel, lanes 2-4). This showed that TIP47 silencing did not influence the synthesis of intracellular Gag and Env proteins (Fig. 2).

We then harvested the virus-containing supernatants of these TIP47-depleted cells and quantified them for CAp24 content. After ultracentrifugation, we used western blotting with antibodies against TMgp41 and CAp24 to evaluate their Gag and Env protein content compared to virions produced from cells treated with siRNA Luc. We found similar levels of Gag protein in all virus preparations (Fig 2A, lower panel and Fig. 2B), whereas the level of TMgp41 in virions produced from TIP47-depleted cells was much lower than in control virions produced from cells treated with siRNA Luc (Fig. 2A, lower panel, lanes 3-4 vs lane 2). This defect in Env incorporation was correlated with low virion infectivity (Fig. 2C). We excluded the possibility that the activity of TIP47 siRNA was due to an off-target effect by checking whether the effect of TIP47 siRNA could be suppressed by re-expression of the HA-TIP-47 construct resistant to siRNA TIP-A (see Fig. 2A lane 5). The siRNA TIP-A targeted a sequence in the 5'UTR of TIP47 cDNA that is not present in the HA-TIP47 construct containing only the TIP47 ORF. Transcomplementation of TIP47-depleted cells with HA-TIP47 (Fig. 2A, lower panel, lane 5) resulted in TMgp41 (Env subunit) being incorporated into virions at a similar level to that seen in cells treated with the control siRNA (lane 2). Moreover, the expression of HA-TlP47 in TIP47-depleted cells almost completely restored the infectivity of the produced virions (Fig. 2C).

Altogether, these results support the conclusion that TIP47 is essential for Env incorporation into virions.

### EXAMPLE 3 : Overexpression of TIP47 enhances Env incorporation into virions

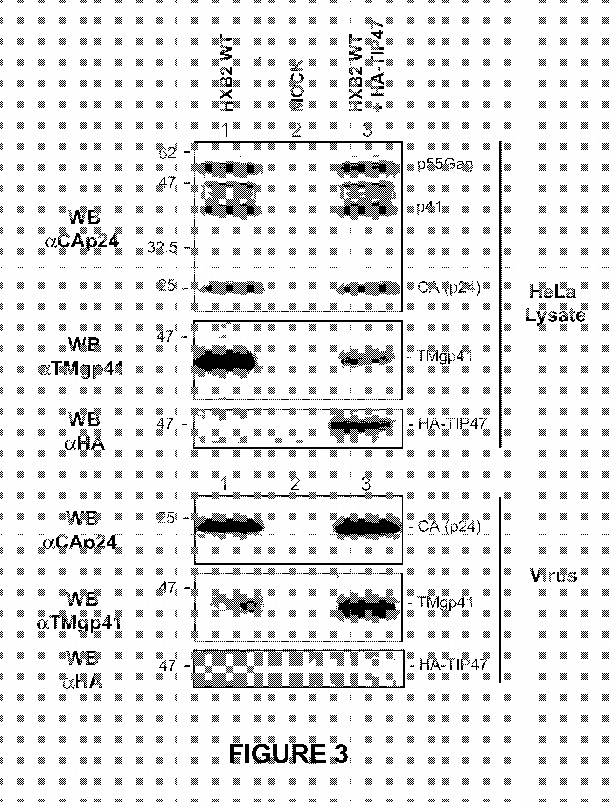

We next investigated whether overexpression of TIP47 could increase the amount of Env incorporated into virions. We produced HIV-1 viruses from cell transiently overexpressing HA-TIP47 by transfecting HeLa cells with HIV-1 HXB2R proviral DNA and a vector expressing HA-TIP47. We analysed virus-producing HeLa cells and ultracentrifuged virus-containing supernatants for Gag and Env proteins by immunoblotting with anti-CA and anti-TMgp41 antibodies (Fig. 3). We detected similar levels of Gag protein in cells overexpressing HA-TIP47 as in cells transfected with the control vector alone (upper panel, lane 3 vs 1). By contrast, intracellular levels of TMgp41 were three times lower in cells overexpressing HA-TIP47 than in control cells (upper panel, lane 3 vs 1). This decrease in the intracellular levels of TMgp41 correlated with a substantial increase in the level of TMgp41 incorporated into virions. Indeed, the level of TMgp41 in virions produced from Hela cells overexpressing HA-TIP47 was estimated as 3.3 times higher than in the virus produced in absence of HA-TIP47 (lower panel, lane 3 *vs* 1), whereas the levels of CAp24 were similar in both types of virion. This showed that overexpression of TlP47 can enhance Env incorporation into virions.

Interestingly, we could not detect any HA-TIP in the virions produced from cells overexpressing HA-TIP47 (lower panel, lane 3) despite there being a moderately high level of the overexpressed protein. This suggests that TIP is not incorporated into virion particles despite its role in Env incorporation into virions.

### EXAMPLE 4 : MAp18 interacts with TIP47

As TIP47 expression was required for producing infectious HIV virions, and as TIP47 interacts with Env through an Y₈₀₂W₈₀₃ motif located in the TMgp41 tail, as previously reported (Blot *et al.,* 2003, *Supra),* we wondered whether TIP47 physically connected Env to Gag to promote Env incorporation into Gag particles. Therefore, we investigated whether TIP47 could interact with Pr55Gag by carrying out co-immunoprecipation assays between Gag protein and TIP47. In cells co-transfected with HA-TIP47 and HXB2R ΔEnv proviral DNA , HA-TIP47 efficiently co-precipitated the Pr55Gag precursor as well as the p41 maturation product (an intermediate cleavage product corresponding to the MA domain linked to CA, Fig. 4A, lower panel, lane 4). Interestingly, the CA domain, which is abundant in transfected HeLa cell lysate, was not precipitated with HA-TlP47. This shows that Gag binds to TIP47 and suggests that the Gag-TlP47 interaction may require the MA domain present in both Pr55Gag and p41 Gag.

We next used a yeast two-hybrid assay to characterize the Gag precursor domain involved in Gag-TIP47 interaction (Fig. 4B). Individual fragments representing MA, CA and MA-CA p41 products were fused at the N-terminus of the LexA-binding domain and tested for interaction with TlP47. Only the MA containing constructs, MAp18 and MA-CA p41, bound efficiently to TlP47 indicating that the MA domain is essential for TlP47 binding. We confirmed the interaction between the MA domain and TlP47 using an *in vitro* TlP47 binding assay (Fig. 4C). HA-TlP47 bound to MA (lane 4) but not to GST (lane 2) or to CA (lane 5). HA-TIP47 also interacts with itself (TlP47, lane 6) as previously shown (Sincock, P. M., Ganley, I. G., Krise, J. P., Diederichs, S., Sivars, U., O'Connor, B., Ding, L., and Pfeffer, S. R. (2003). Self-assembly is important for TlP47 function in mannose 6-phosphate receptor transport. Traffic 4, 18-25) and with the TMgp41 CD (Env HIV, lane 3) as previously described by us (Blot *et al.,* 2003, *Supra).*

These results demonstrate that TIP47 can physically interact both with Env, through the TMgp41 cytoplasmic tail, and with Gag, through the MA domain.

### EXAMPLE 5 : The residues 5 to 16 of MA are required for the binding to TIP47

We used several deleted constructs of MA lacking the C-terminal part to determine precisely the MA region required for the MA-TIP47 interaction. We then used yeast two-hybrid assay to analyse their ability to bind to TIP47. These deletion studies showed that the first 23 amino acids of the MA domain are sufficient to mediate the interaction with TIP47 (Fig. 4D). We next carried out site-directed mutational analysis with Ala scanning residues two-by-two on the 23 first amino acids of the MA domain to identify the residues involved in the TIP47 interaction (Fig. 5A). We checked that all the mutants were similarly expressed in yeast extract (Fig. 5A, lower panel). A quantitative two-hybrid assay of TIP47 binding to MA₁₋₂₃ mutants showed that the sequence that contained the amino acids 5 to 16 of MA was involved in the MA-TIP47 interaction. The peptide fragments S₅-VL-S₈ and D₁₃-RW-E₁₆ appeared to be essential for MA to TIP47 binding (Fig. 5A).

We then introduced mutations to residues S₅V₆ and W₁₅E₁₆ in the HXB2R proviral DNA to analyse the involvement of these residues in the MA-TIP47 interaction in infected cells. We immunoprecipitated lysates of HeLa cells expressing HA-tagged TIP47 and one of either wild type proviral HIV DNA or MA S₅V₆-AA, MA W₁₅E₁₆-AA or Env Y₈₀₂W₈₀₃-SL (previously described in Blot *et al.* 2003, Supra)-mutated HIV DNAs with an anti-HA antibody and detected Gag products in these immunoprecipitates with an anti-p24 Gag antibody. The Pr55Gag precursor co-immunoprecipitated with HA-TIP47 in lysates of cells expressing the Gag precursor alone (HXB2 ΔEnv) (Fig. 5B, lower panels, lane 7), the HIV-1 HXB2R wt (HXB2) (lane 8) or the Env Y₈₀₂W₈₀₃-SL mutated HIV-1 (HXB2 YW-SL, a virus having an envelope glycoprotein that cannot interact with TIP47) (lane 10). By contrast, we observed no association of the Gag precursor with HA-TIP47 in cells transfected with HIV-1 proviral DNA that was mutated in residues S₅V₆ (lane 11) or W₁₅E₁₆ (lane 9) of the MA domain. Interestingly, we could not detect the CA product in HXB2R S₅V₆-AA cells lysates (upper panel, CAp24, lanes 6 and 11), showing that introducing the S₅V₆-AA double mutation into the MA domain of HXB2R Gag blocked the intracellular maturation of Pr55Gag, whereas in the W₁₅E₁₆AA MA mutant, we detected the intracellular maturation of Pr55Gag (see upper panel, CAp24, lanes 4 and 9). A similar maturation defect has been previously described in a virus bearing a V₆-R mutation in the MA domain (Ono and Freed, 1999). Thus, there is a clear correlation between the loss of TIP47 binding and MA W₁₅E₁₆-AA mutations in HIV-1.

### EXAMPLE 6 : The MA W₁₅E₁₆-AA mutation results in defect in Env incorporation and production of non-infectious virus.

We then checked the infectivity of the MA W₁₅E₁₆AA mutant virus, a mutant deficient for the binding of Gag to TIP47 and unaffected in intracellular Gag maturation, to try to correlate the absence of the Gag-TIP47 interaction with defects in infectivity and Env incorporation.

We generated wt and MA W₁₅E₁₆-AA HIV-1 viral stocks by transfecting 293T cells with the corresponding HXB2R proviral DNAs. We also produced a control Env Y₈₀₂W₈₀₃-SL mutant HIV-1 viral stock, which cannot replicate in Jurkat T cells as previously shown (Blot *et al.* 2003, *Supra).* We observed the same intracellular level of expression of Gag and Env proteins in 293T cells producing HIV-1 HXB2R wt and the MA W₁₅E₁₆-AA or Env Y₈₀₂W₈₀₃-SL mutants (Fig. 6A). To compare the replication kinetics of these three viruses in Jurkat T cells, we measured the amount of HIV p24 antigen produced in cell culture supernatants during 23 days infection. The HIV-1 wt virus productively infected Jurkat T cells (Fig. 6B), whereas the HIV-1 MAW₁₅E₁₆-AA and EnvY₈₀₂W₈₀₃-SL mutants almost completely failed to replicate in Jurkat cells (Fig. 6B). We observed the same results in HeLa P4-2 cells (data not shown). These results show that the MA W₁₅E₁₆-AA residues involved in the MA-TIP47 interaction play an essential role in optimal HIV-1 infection.

We then analysed the effect of the MA W₁₅E₁₆-AA mutation on Env incorporation into T-cell lines using a high-level, transient HIV-1 expression system based on pseudotyping with VSV-G (Murakami, T., and Freed, E. O. (2000). Genetic evidence for an interaction between human immunodeficiency virus type 1 matrix and alpha-helix 2 of the gp41 cytoplasmic tail. J Virol 74, 3548-3554). We co-transfected 293T cells with a VSV-G expression vector and HXB2R wt proviral DNA or either of the MA W₁₅E₁₆-AA or the Env Y₈₀₂W₈₀₃-SL mutants. We collected the VSV-G-pseudotyped virus stocks and determined the titers of these viral stocks. We then infected Jurkat T cells with these viral stocks at a multiplicity of infection (M.O.I) of 1. Two days after infection, we harvested the cells and the virions were ultracentrifuged. We observed the same level of intracellular expression and processing of Gag and Env proteins in Jurkat T cells producing HIV-1 HXB2R wt, or the MA W₁₅E₁₆-AA or Env Y₈₀₂W₈₀₃-SL mutants (Fig. 6C, upper panels). We detected normal wild-type levels of CAp24 in all the virions in the Jurkat T cell supernatants but only very low levels of Env TMgp41 proteins in the MA W₁₅E₁₆-AA or Env Y₈₀₂W₈₀₃-SL mutant virions (Fig. 6C, lower panel, lanes 3-4 vs 2). The MA W₁₅E₁₆-AA mutated virion produced from HeLa cells also had very low levels of incorporated TMgp41 (Fig. 6D lower panel, lane 3 vs 2), consistent with the results in Jurkat T cells. Although the Env Y₈₀₂W₈₀₃-SL mutant virions from Hela cells had low levels of incorporated TMgp41, it was not as low as that detected in the MA W₁₅E₁₆-AA mutant virions or in both mutant virions from Jurkat cells (Fig. 6D lower panel, lane 4 vs 2). This defect in the incorporation of Env in the virions correlated with a very low virion infectivity (Fig. 6E).

Altogether, these results demonstrate that impairing the interaction of MA or TMgp41 with TIP47 with the respective MA W₁₅E₁₆₋AA or Env Y₈₀₂W₈₀₃-SL mutants results in loss of Env incorporation and virion infectivity.

### EXAMPLE 7 : TIP47 acts as a connector between MA and TMgp41

TIP47 can interact with both the Env TMgp41 subunit and the MA domain of Gag. Therefore, to demonstrate that these TlP47 interactions can indeed act as a true connector between Gag and Env, we examined the effect of TIP47 depletion on the binding of Env to Gag in HeLa cells producing HIV-1 virions after transfection with HXB2R proviral DNA. We immunoprecipitated the lysates of transfected HeLa cells with the TMgp41 antibody (Fig. 7). Gag efficiently co-immunoprecipitated with TMgp41 in untreated cells (lane 2) or in cells treated with Luc siRNA (lane 3) that expressed the HXB2R provirus, whereas in cells in which TIP47 expression was silenced by treatment with TIP-A siRNA, the association of Gag to TMgp41 was considerably reduced (lower panel, lanes 2 and 3 vs 4).

These results demonstrate that TIP47 expression is needed for the association of Gag with the Env TMgp41 subunit in virion-producing cells, which suggests that TIP47 can act as an effective connector between Gag and Env.

### Example 8 : HTRF assay

An amount of 250 ng of MA-His6x was incubated in buffer A containing Anti-His-Cryptate and Anti-GST-XL665 (Cisbio) antibodies with various quantities of GST-TIP47 (900ng, 450 ng, 225 ng, 112.5ng, 56ng, 28ng, 14ng, 7ng, 3.5ng, 1.75ng, 0.875ng).

A detectable fluorescence signal at 665 nm was measured for amounts of GST-TIP47 ranging from 112.5 ng to 1.75 ng. The strongest intensity of the fluorescence signal was measured for amounts of GST-TIP47 ranging from 56 ng to 28 ng.

No signal was detected in presence of GST alone or in presence of His-MA purified protein presenting of His6x tag in N-terminus of MA domain.

The main sequences disclosed herein are summarized in Table 1 hereunder.

**Table 1**

| **SEQ ID N°** | **Type** | **Description** |
|---|---|---|
| 1 | protein | minimal HIV-1 Gag-derived peptide |
| 2 | protein | TIP47 cellular protein |
| 3 | protein | complete HIV-1 Gag protein |
| 4 | nucleic acid | HXB2 HIV-1 proviral DNA |
| 5 | nucleic acid | nucleic acid encoding TIP47 |
| 6 | protein | variable minimal HIV-1 Gag-derived peptide |
| 7 | protein | variable extended minimal HIV-1 Gag-derived peptide |

## Claims

1. A method for the *in vitro* screening of compounds that inhibit Env glycoprotein incorporation into HIV-1 virions in cells infected by a HIV-1 virus, wherein said method comprises the following steps
a) determining the ability of a candidate compound to inhibit the interaction between (i) the HIV-1 Gag protein and (ii) the TIP47 protein; and
b) selecting positively the candidate compound(s) that inhibit the interaction between (i) the HIV-1 Gag protein and (ii) the TIP47 protein.

2. The method according to claim 1, wherein step a) consists of the following steps :
a1) bringing into contact the candidate compound to be tested with a target polypeptide selected from the group consisting of:
(1) a first polypeptide comprising an amino acid sequence selected from the group consisting of :
(A) the peptide consisting the amino acid sequence of SEQ lD N° 6 below :
NH₂-S-X₄-L-X₅-X₆-X₇-X₈-X₉-X₁₀-X₁₁-W-E- COOH, wherein
- X4 is an amino acid residue selected from the group consisting of V, L, I, T, R and E;
- X5 is an amino acid residue selected from the group consisting of S, T, R, I and K;
- X6 is an amino acid residue selected from the group consisting of G and A;
- X7 is an amino acid residue selected from the group consisting of G,R,K,E,S,T,KandD;
- X8 is an amino acid residue selected from the group consisting of E, K, R, Q and G;
- X9 is an amino acid residue selected from the group consisting of L, F, K and Q;
- X10 is an amino acid residue selected from the group consisting of D and E; and
- X11 is an amino acid residue selected from the group consisting of R, A, S, K, Q, T, E, N, D
(B) the peptide consisting the amino acid sequence of SEQ ID N° 7 below :
NH₂-X₁-X₂-A-X₃-A-S-X₄-L-X₅-X₆-X₇-X₈-X₉-X₁₀-X₁₁-W-E-X₁₂-X₁₃-X₁₄-X₁₅-X₁₆-P-COOH, wherein
- X1 is an amino acid residue selected from the group consisting of M, L, V and I;
- X2 is an amino acid residue selected from the group consisting of G, S and T;
- X3 is an amino acid residue selected from the group consisting of R, G, S and K;
- X4 is an amino acid residue selected from the group consisting of V, L, I, T, R and E;
- X5 is an amino acid residue selected from the group consisting of S, T, R, I and K;
- X6 is an amino acid residue selected from the group consisting of G and A;
- X7 is an amino acid residue selected from the group consisting of G,R,K,E,S,T,KandD;
- X8 is an amino acid residue selected from the group consisting of E, K, R, Q and G;
- X9 is an amino acid residue selected from the group consisting of L, F, K and Q;
- X10 is an amino acid residue selected from the group consisting of D and E;
- X11 is selected from the group consisting of R, A, S, K, Q, T, E, N, D and P;
- X12 is an amino acid residue selected from the group consisting of K, R, N, E, A and S;
- X13 is an amino acid residue selected from the group consisting of I, M, Q and T;
- X14 is an amino acid residue selected from the group consisting of R, K, W, Q, H and Y;
- X15 is an amino acid residue selected from the group consisting of L and S; and
- X16 is an amino acid residue selected from the group consisting of R and K. and
(C) a peptide having at least 60% amino acid identity with the Gag amino acid sequence of SEQ ID N°1;
or
(2) a second polypeptide comprising the amino acid sequence of SEQ lD N°2 or a peptide fragment thereof;
a2) determining the ability of said candidate compound to bind to said target polypeptide;

3. The method according to claim 2, wherein said first polypeptide comprises or consists of the polypeptide of SEQ ID N° 1.

4. The method according to claim 2, wherein said second polypeptide consists of the polypeptide of SEQ ID N° 2 or a peptide fragment thereof.

5. The method according to any one of claims 2 to 4, wherein said first or second polypeptide is labelled with a detectable molecule.

6. The method according to any one of claims 2 to 5, wherein said candidate compound is labelled with a detectable molecule.

7. The method according to claim 1, wherein step a) consists of the following steps :
a1) bringing into contact the candidate compound to be tested with a mixture of
(1) a first polypeptide comprising an amino acid sequence selected from the group consisting of (i) the amino acid sequence of SEQ ID N°6, (ii) the amino acid sequence of SEQ lD N°7 and (iii) an amino acid sequence having at least 60% amino acid identity with the amino acid of SEQ ID N°1, and
(2) a second polypeptide comprising the amino acid sequence of SEQ ID N°2 or a peptide fragment thereof;
a2) determining the ability of said candidate compound to inhibit the binding between said first polypeptide and said second polypeptide.

8. The method according to claim 7, wherein said first polypeptide comprises or consists of the polypeptide of SEQ ID N° 1.

9. The method according to claim 7, wherein said second polypeptide consists of the polypeptide of SEQ ID N° 2 or a peptide fragment thereof.

10. The method according to any one claims 7 to 9, wherein step a2) consists of measuring the ability of said candidate compound to inhibit co-precipitation of said first and second polypeptides.

11. The method according to any one of claims 7 to 9, wherein :
- the first polypeptide is fused to a first portion of a transcription activator protein;
- the second polypeptide is fused to a second portion of a transcription activation factor,
wherein the binding of the first and second portions of said transcription factor results in the formation of an active transcription factor.

12. The method according to claim 10, wherein :
- the first polypeptide is fused to the LexA binding domain;
- the second polypeptide is fused to the Gal4 activator domain,
wherein the binding of LexA to Gal4 results in the formation of a transcription factor that induces the expression of a reporter gene comprising the LexA regulatory polynucleotide.

13. The method according to any one of claims 7 to 9, wherein :
- the first polypeptide is labelled with a detectable molecule; and/or
- the second polypeptide is labelled with a detectable molecule.

14. The method according to any one of claims 7 to 9, wherein :
- the first polypeptide is labelled with a first fluorophore substance;
- the second polypeptide is labelled with a second fluorophore substance.

15. The method according to claim 14, wherein :
- the first fluorophore substance has an emission wavelength value sensibly equal to the excitation wavelength value of the second fluorophore substance; and
- the binding of said first and second polypeptides is detected by measuring the fluorescence signal intensity emitted at the emission wavelength value of the second fluorophore substance.

16. The method according to claim 14, wherein :
- the second fluorophore substance has an emission wavelength value sensibly equal to the excitation wavelength value of the first fluorophore; and
- the binding of said first and second polypeptides is detected by measuring the fluorescence signal intensity emitted at the emission wavelength value of the second fluorophore substance.

17. The method according to any one of claims 14 to 16, wherein :
- one polypeptide selected from the group consisting of said first and second polypeptides is labelled with Europium cryptate compound, whereas
- the other polypeptide is labelled with a cross-linked allophycocyanin compound; and
- the binding of said first and second polypeptides is detected by measuring the fluorescence signal intensity emitted at the emission wavelength value of said cross-linked phycobiliprotein.

18. A method for the screening of compounds that inhibit formation of infectious HIV-1 virions within cells infected with HIV-1 virus, wherein said method comprises the steps of :
i) screening for compounds that inhibit Env glycoprotein incorporation into HIV-1 virions in cells infected by a HIV-1 virus, by performing the screening method according to any one of claims 1 to 17; and
ii) screening the compounds positively selected at the end of step i) for their ability to inhibit formation of infectious HIV-1 virions within cells infected with HIV-1 virus.

19. The method of claim 18, wherein step ii) is selected from the group consisting of the following steps ii):
A)
ii-1) infecting cultured mammalian cells with HIV-1 virus;
ii-2) bringing into contact the infected cells obtained at step ii-1) with a compound that has been positively selected at the end of step i);
ii-3) determining the infectious capacity of the HIV virions produced by the said infected mammalian cells; and
ii-4) comparing the infectious capacity of the HIV virions determined at step ii-3) with the infectious capacity of the HIV virions that is determined when step ii-2) is performed in the absence of the said positively selected compound,
or
B)
ii-2) bringing into contact cultured mammalian cells with a compound that has been positively selected at the end of step i);
ii-1) infecting the cultured mammalian obtained at step ii-2) with HIV-1 virus;
ii-3) determining the infectious capacity of the HIV virions produced by the said infected mammalian cells; and
ii-4) comparing the infectious capacity of the HIV virions determined at step ii-3) with the infectious capacity of the HIV virions that is determined when step ii-2) is performed in the absence of the said positively selected compound.

20. A polypeptide that inhibits infectious HIV-1 virions production in mammalian HIV-1 infected cells, comprising a Gag-derived peptide sequence selected from the group consisting of (i) the amino acid sequence of SEQ ID N°6, (ii) the amino acid sequence of SEQ ID N°7 and (iii) an amino acid sequence having at least 60% amino acid identity with the amino acid of SEQ ID N°1

21. The polypeptide according to claim 20 consisting of the amino acid sequence of SEQ ID N°1.

22. A composition comprising the polypeptide according to any one of claims 19 and 20 in combination with one or more auxiliary substances.

23. A pharmaceutical composition comprising the polypeptide according to any one of claims 20 and 21 in combination with one or more physiologically acceptable excipients.

24. The use of a polypeptide according to any one of claims 20 and 21 for manufacturing a pharmaceutical composition for preventing or treating a disease linked with the infection of an individual with a virus of the HIV family.


SEQUENCE LISTING

<110>  Institut National de la Sante et de la Recherche Medicale (INSERM)

<120>  Methods for the in vitro screening of compounds inhibiting production of
infectious HIV-1 virions and compounds selected by said method

<130>  U040-EP

<160>  7

<170>  PatentIn version 3.1

<210>  1
<211>  12
<212>  PRT
<213>  Human immunodeficiency virus type 1

<400>  1

Ser Val Leu Ser Gly Gly Glu Leu Asp Arg Trp Glu
1               5                   10

<210>  2
<211>  434
<212>  PRT
<213>  Homo sapiens

<400>  2

Met Ser Ala Asp Gly Ala Glu Ala Asp Gly Ser Thr Gln Val Thr Val
1               5                   10                  15

Glu Glu Pro Val Gln Gln Pro Ser Val Val Asp Arg Val Ala Ser Met
            20                  25                  30

Pro Leu Ile Ser Ser Thr Cys Asp Met Val Ser Ala Ala Tyr Ala Ser
        35                  40                  45

Thr Lys Glu Ser Tyr Pro His Val Lys Thr Val Cys Asp Ala Ala Glu
    50                  55                  60

Lys Gly Val Arg Thr Leu Thr Ala Ala Ala Val Ser Gly Ala Gln Pro
65                  70                  75                  80

Ile Leu Ser Lys Leu Glu Pro Gln Ile Ala Ser Ala Ser Glu Tyr Ala
                85                  90                  95

His Arg Gly Leu Asp Lys Leu Glu Glu Asn Leu Pro Ile Leu Gln Gln
            100                 105                 110

Pro Thr Glu Lys Val Leu Ala Asp Thr Lys Glu Leu Val Ser Ser Lys
        115                 120                 125

Val Ser Gly Ala Gln Glu Met Val Ser Ser Ala Lys Asp Thr Val Ala
    130                 135                 140

Thr Gln Leu Ser Glu Ala Val Asp Ala Thr Arg Gly Ala Val Gln Ser
145                 150                 155                 160

Gly Val Asp Lys Thr Lys Ser Val Val Thr Gly Gly Val Gln Ser Val
                165                 170                 175

Met Gly Ser Arg Leu Gly Gln Met Val Leu Ser Gly Val Asp Thr Val
            180                 185                 190

Leu Gly Lys Ser Glu Glu Trp Ala Asp Asn His Leu Pro Leu Thr Asp
        195                 200                 205

Ala Glu Leu Ala Arg Ile Ala Thr Ser Leu Asp Gly Phe Asp Val Ala
    210                 215                 220

Ser Val Gln Gln Gln Arg Gln Glu Gln Ser Tyr Phe Val Arg Leu Gly
225                 230                 235                 240

Ser Leu Ser Glu Arg Leu Arg Gln His Ala Tyr Glu His Ser Leu Gly
                245                 250                 255

Lys Leu Arg Ala Thr Lys Gln Arg Ala Gln Glu Ala Leu Leu Gln Leu
            260                 265                 270

Ser Gln Ala Leu Ser Leu Met Glu Thr Val Lys Gln Gly Val Asp Gln
        275                 280                 285

Lys Leu Val Glu Gly Gln Glu Lys Leu His Gln Met Trp Leu Ser Trp
    290                 295                 300

Asn Gln Lys Gln Leu Gln Gly Pro Glu Lys Glu Pro Pro Lys Pro Glu
305                 310                 315                 320

Gln Val Glu Ser Arg Ala Leu Thr Met Phe Arg Asp Ile Ala Gln Gln
                325                 330                 335

Leu Gln Ala Thr Cys Thr Ser Leu Gly Ser Ser Ile Gln Gly Leu Pro

340                    345                    350

Thr Asn Val Lys Asp Gln Val Gln Gln Ala Arg Arg Gln Val Glu Asp
        355                 360                 365

Leu Gln Ala Thr Phe Ser Ser Ile His Ser Phe Gln Asp Leu Ser Ser
    370                 375                 380

Ser Ile Leu Ala Gln Ser Arg Glu Arg Val Ala Ser Ala Arg Glu Ala
385                 390                 395                 400

Leu Asp His Met Val Glu Tyr Val Ala Gln Asn Thr Pro Val Thr Trp
                405                 410                 415

Leu Val Gly Pro Phe Ala Pro Gly Ile Thr Glu Lys Ala Pro Glu Glu
            420                 425                 430

Lys Lys

<210>  3
<211>  500
<212>  PRT
<213>  Human immunodeficiency virus type 1

<400>  3

Met Gly Ala Arg Ala Ser Val Leu Ser Gly Gly Glu Leu Asp Arg Trp
1               5                   10                  15

Glu Lys Ile Arg Leu Arg Pro Gly Gly Lys Lys Lys Tyr Lys Leu Lys
            20                  25                  30

His Ile Val Trp Ala Ser Arg Glu Leu Glu Arg Phe Ala Val Asn Pro
        35                  40                  45

Gly Leu Leu Glu Thr Ser Glu Gly Cys Arg Gln Ile Leu Gly Gln Leu
    50                  55                  60

Gln Pro Ser Leu Gln Thr Gly Ser Glu Glu Leu Arg Ser Leu Tyr Asn
65                  70                  75                  80

Thr Val Ala Thr Leu Tyr Cys Val His Gln Arg Ile Glu Ile Lys Asp
                85                  90                  95

Thr Lys Glu Ala Leu Asp Lys Ile Glu Glu Glu Gln Asn Lys Ser Lys
            100                 105                 110

Lys Lys Ala Gln Gln Ala Ala Ala Asp Thr Gly His Ser Asn Gln Val
        115                 120                 125

Ser Gln Asn Tyr Pro Ile Val Gln Asn Ile Gln Gly Gln Met Val His
    130                 135                 140

Gln Ala Ile Ser Pro Arg Thr Leu Asn Ala Trp Val Lys Val Val Glu
145                 150                 155                 160

Glu Lys Ala Phe Ser Pro Glu Val Ile Pro Met Phe Ser Ala Leu Ser
                165                 170                 175

Glu Gly Ala Thr Pro Gln Asp Leu Asn Thr Met Leu Asn Thr Val Gly
            180                 185                 190

Gly His Gln Ala Ala Met Gln Met Leu Lys Glu Thr Ile Asn Glu Glu
        195                 200                 205

Ala Ala Glu Trp Asp Arg Val His Pro Val His Ala Gly Pro Ile Ala
    210                 215                 220

Pro Gly Gln Met Arg Glu Pro Arg Gly Ser Asp Ile Ala Gly Thr Thr
225                 230                 235                 240

Ser Thr Leu Gln Glu Gln Ile Gly Trp Met Thr Asn Asn Pro Pro Ile
                245                 250                 255

Pro Val Gly Glu Ile Tyr Lys Arg Trp Ile Ile Leu Gly Leu Asn Lys
            260                 265                 270

Ile Val Arg Met Tyr Ser Pro Thr Ser Ile Leu Asp Ile Arg Gln Gly
        275                 280                 285

Pro Lys Glu Pro Phe Arg Asp Tyr Val Asp Arg Phe Tyr Lys Thr Leu
    290                 295                 300

Arg Ala Glu Gln Ala Ser Gln Glu Val Lys Asn Trp Met Thr Glu Thr
305                 310                 315                 320

Leu Leu Val Gln Asn Ala Asn Pro Asp Cys Lys Thr Ile Leu Lys Ala
                325                 330                 335

Leu Gly Pro Ala Ala Thr Leu Glu Glu Met Met Thr Ala Cys Gln Gly
            340                 345                 350

Val Gly Gly Pro Gly His Lys Ala Arg Val Leu Ala Glu Ala Met Ser
        355                 360                 365

Gln Val Thr Asn Ser Ala Thr Ile Met Met Gln Arg Gly Asn Phe Arg
    370                 375                 380

Asn Gln Arg Lys Ile Val Lys Cys Phe Asn Cys Gly Lys Glu Gly His
385                 390                 395                 400

Thr Ala Arg Asn Cys Arg Ala Pro Arg Lys Lys Gly Cys Trp Lys Cys
                405                 410                 415

Gly Lys Glu Gly His Gln Met Lys Asp Cys Thr Glu Arg Gln Ala Asn
            420                 425                 430

Phe Leu Gly Lys Ile Trp Pro Ser Tyr Lys Gly Arg Pro Gly Asn Phe
        435                 440                 445

Leu Gln Ser Arg Pro Glu Pro Thr Ala Pro Pro Glu Glu Ser Phe Arg
    450                 455                 460

Ser Gly Val Glu Thr Thr Thr Pro Pro Gln Lys Gln Glu Pro Ile Asp
465                 470                 475                 480

Lys Glu Leu Tyr Pro Leu Thr Ser Leu Arg Ser Leu Phe Gly Asn Asp
                485                 490                 495

Pro Ser Ser Gln
            500

<210>  4

<211>  9719

<212>  DNA

<213>  Human immunodeficiency virus type 1

<400>  4
tggaagggct aattcactcc caacgaagac aagatatcct tgatctgtgg atctaccaca      60

cacaaggcta cttccctgat tagcagaact acacaccagg gccagggatc agatatccac     120

tgacctttgg atggtgctac aagctagtac cagttgagcc agagaagtta gaagaagcca     180

acaaaggaga gaacaccagc ttgttacacc ctgtgagcct gcatggaatg gatgacccgg     240

agagagaagt gttagagtgg aggtttgaca gccgcctagc atttcatcac atggcccgag     300

agctgcatcc ggagtacttc aagaactgct gacatcgagc ttgctacaag ggactttccg     360

ctggggactt tccagggagg cgtggcctgg gcgggactgg ggagtggcga gccctcagat     420

cctgcatata agcagctgct ttttgcctgt actgggtctc tctggttaga ccagatctga     480

gcctgggagc tctctggcta actagggaac ccactgctta agcctcaata aagcttgcct     540

tgagtgcttc aagtagtgtg tgcccgtctg ttgtgtgact ctggtaacta gagatccctc     600

agaccctttt agtcagtgtg gaaaatctct agcagtggcg cccgaacagg gacctgaaag     660

cgaaagggaa accagaggag ctctctcgac gcaggactcg gcttgctgaa gcgcgcacgg     720

caagaggcga ggggcggcga ctggtgagta cgccaaaaat tttgactagc ggaggctaga     780

aggagagaga tgggtgcgag agcgtcagta ttaagcgggg gagaattaga tcgatgggaa     840

aaaattcggt taaggccagg gggaaagaaa aaatataaat taaaacatat agtatgggca     900

agcagggagc tagaacgatt cgcagttaat cctggcctgt tagaaacatc agaaggctgt 960
agacaaatac tgggacagct acaaccatcc cttcagacag gatcagaaga acttagatca 1020
ttatataata cagtagcaac cctctattgt gtgcatcaaa ggatagagat aaaagacacc 1080
aaggaagctt tagacaagat agaggaagag caaaacaaaa gtaagaaaaa agcacagcaa 1140
gcagcagctg acacaggaca cagcaatcag gtcagccaaa attaccctat agtgcagaac 1200
atccaggggc aaatggtaca tcaggccata tcacctagaa ctttaaatgc atgggtaaaa 1260
gtagtagaag agaaggcttt cagcccagaa gtgataccca tgttttcagc attatcagaa 1320
ggagccaccc cacaagattt aaacaccatg ctaaacacag tggggggaca tcaagcagcc 1380
atgcaaatgt taaaagagac catcaatgag gaagctgcag aatgggatag agtgcatcca 1440
gtgcatgcag ggcctattgc accaggccag atgagagaac caaggggaag tgacatagca 1500
ggaactacta gtacccttca ggaacaaata ggatggatga caaataatcc acctatccca 1560
gtaggagaaa tttataaaag atggataatc ctgggattaa ataaaatagt aagaatgtat 1620
agccctacca gcattctgga cataagacaa ggaccaaagg aaccctttag agactatgta 1680
gaccggttct ataaaactct aagagccgag caagcttcac aggaggtaaa aaattggatg 1740
acagaaacct tgttggtcca aaatgcgaac ccagattgta agactatttt aaaagcattg 1800
ggaccagcgg ctacactaga agaaatgatg acagcatgtc agggagtagg aggacccggc 1860
cataaggcaa gagttttggc tgaagcaatg agccaagtaa caaattcagc taccataatg 1920
atgcagagag gcaattttag gaaccaaaga aagattgtta agtgtttcaa ttgtggcaaa 1980
gaagggcaca cagccagaaa ttgcagggcc cctaggaaaa agggctgttg gaaatgtgga 2040
aaggaaggac accaaatgaa agattgtact gagagacagg ctaatttttt agggaagatc 2100
tggccttcct acaagggaag gccagggaat tttcttcaga gcagaccaga gccaacagcc 2160
ccaccagaag agagcttcag gtctggggta gagacaacaa ctccccctca gaagcaggag 2220
ccgatagaca aggaactgta tcctttaact tccctcaggt cactctttgg caacgacccc 2280
tcgtcacaat aaagataggg gggcaactaa aggaagctct attagataca ggagcagatg 2340
atacagtatt agaagaaatg agtttgccag gaagatggaa accaaaaatg atagggggaa 2400
ttggaggttt tatcaaagta agacagtatg atcagatact catagaaatc tgtggacata 2460
aagctatagg tacagtatta gtaggaccta cacctgtcaa cataattgga agaaatctgt 2520
tgactcagat tggttgcact ttaaattttc ccattagccc tattgagact gtaccagtaa 2580
aattaaagcc aggaatggat ggcccaaaag ttaaacaatg gccattgaca gaagaaaaaa 2640
taaaagcatt agtagaaatt tgtacagaga tggaaaagga agggaaaatt tcaaaaattg 2700
ggcctgaaaa tccatacaat actccagtat ttgccataaa gaaaaaagac agtactaaat 2760
ggagaaaatt agtagatttc agagaactta ataagagaac tcaagacttc tgggaagttc 2820
aattaggaat accacatccc gcagggttaa aaaagaaaaa atcagtaaca gtactggatg 2880
tgggtgatgc atatttttca gttcccttag atgaagactt caggaagtat actgcattta 2940

ccatacctag tataaacaat gagacaccag ggattagata tcagtacaat gtgcttccac 3000
agggatggaa aggatcacca gcaatattcc aaagtagcat gacaaaaatc ttagagcctt 3060
ttagaaaaca aaatccagac atagttatct atcaatacat ggatgatttg tatgtaggat 3120
ctgacttaga aatagggcag catagaacaa aaatagagga gctgagacaa catctgttga 3180
ggtggggact taccacacca gacaaaaaac atcagaaaga acctccattc ctttggatgg 3240
gttatgaact ccatcctgat aaatggacag tacagcctat agtgctgcca gaaaaagaca 3300
gctggactgt caatgacata cagaagttag tggggaaatt gaattgggca agtcagattt 3360
acccagggat taaagtaagg caattatgta aactccttag aggaaccaaa gcactaacag 3420
aagtaatacc actaacagaa gaagcagagc tagaactggc agaaaacaga gagattctaa 3480
aagaaccagt acatggagtg tattatgacc catcaaaaga cttaatagca gaaatacaga 3540
agcaggggca aggccaatgg acatatcaaa tttatcaaga gccatttaaa aatctgaaaa 3600
caggaaaata tgcaagaatg aggggtgccc acactaatga tgtaaaacaa ttaacagagg 3660
cagtgcaaaa aataaccaca gaaagcatag taatatgggg aaagactcct aaatttaaac 3720
tgcccataca aaaggaaaca tgggaaacat ggtggacaga gtattggcaa gccacctgga 3780
ttcctgagtg ggagtttgtt aatacccctc ccttagtgaa attatggtac cagttagaga 3840
aagaacccat agtaggagca gaaaccttct atgtagatgg ggcagctaac agggagacta 3900
aattaggaaa agcaggatat gttactaata gaggaagaca aaaagttgtc accctaactg 3960
acacaacaaa tcagaagact gagttacaag caatttatct agctttgcag gattcgggat 4020
tagaagtaaa catagtaaca gactcacaat atgcattagg aatcattcaa gcacaaccag 4080
atcaaagtga atcagagtta gtcaatcaaa taatagagca gttaataaaa aaggaaaagg 4140
tctatctggc atgggtacca gcacacaaag gaattggagg aaatgaacaa gtagataaat 4200
tagtcagtgc tggaatcagg aaagtactat ttttagatgg aatagataag gcccaagatg 4260
aacatgagaa atatcacagt aattggagag caatggctag tgattttaac ctgccacctg 4320
tagtagcaaa agaaatagta gccagctgtg ataaatgtca gctaaaagga gaagccatgc 4380
atggacaagt agactgtagt ccaggaatat ggcaactaga ttgtacacat ttagaaggaa 4440
aagttatcct ggtagcagtt catgtagcca gtggatatat agaagcagaa gttattccag 4500
cagaaacagg gcaggaaaca gcatattttc ttttaaaatt agcaggaaga tggccagtaa 4560
aaacaataca tactgacaat ggcagcaatt tcaccggtgc tacggttagg gccgcctgtt 4620
ggtgggcggg aatcaagcag gaatttggaa ttccctacaa tccccaaagt caaggagtag 4680
tagaatctat gaataaagaa ttaaagaaaa ttataggaca ggtaagagat caggctgaac 4740
atcttaagac agcagtacaa atggcagtat tcatccacaa ttttaaaaga aaagggggga 4800
ttggggggta cagtgcaggg gaaagaatag tagacataat agcaacagac atacaaacta 4860
aagaattaca aaaacaaatt acaaaaattc aaaattttcg ggtttattac agggacagca 4920
gaaatccact ttggaaagga ccagcaaagc tcctctggaa aggtgaaggg gcagtagtaa 4980
tacaagataa tagtgacata aaagtagtgc caagaagaaa agcaaagatc attagggatt 5040

atggaaaaca gatggcaggt gatgattgtg tggcaagtag acaggatgag gattagaaca 5100
tggaaaagtt tagtaaaaca ccatatgtat gtttcaggga aagctagggg atggttttat 5160
agacatcact atgaaagccc tcatccaaga ataagttcag aagtacacat cccactaggg 5220
gatgctagat tggtaataac aacatattgg ggtctgcata caggagaaag agactggcat 5280
ttgggtcagg gagtctccat agaatggagg aaaaagagat atagcacaca agtagaccct 5340
gaactagcag accaactaat tcatctgtat tactttgact gtttttcaga ctctgctata 5400
agaaaggcct tattaggaca catagttagc cctaggtgtg aatatcaagc aggacataac 5460
aaggtaggat ctctacaata cttggcacta gcagcattaa taacaccaaa aaagataaag 5520
ccacctttgc ctagtgttac gaaactgaca gaggatagat ggaacaagcc ccagaagacc 5580
aagggccaca gagggagcca cacaatgaat ggacactaga gcttttagag gagcttaaga 5640
atgaagctgt tagacatttt cctaggattt ggctccatgg cttagggcaa catatctatg 5700
aaacttatgg ggatacttgg gcaggagtgg aagccataat aagaattctg caacaactgc 5760
tgtttatcca ttttcagaat tgggtgtcga catagcagaa taggcgttac tcgacagagg 5820
agagcaagaa atggagccag tagatcctag actagagccc tggaagcatc caggaagtca 5880
gcctaaaact gcttgtacca attgctattg taaaaagtgt tgctttcatt gccaagtttg 5940
tttcataaca aaagccttag gcatctccta tggcaggaag aagcggagac agcgacgaag 6000
agctcatcag aacagtcaga ctcatcaagc ttctctatca aagcagtaag tagtacatgt 6060
aacgcaacct ataccaatag tagcaatagt agcattagta gtagcaataa taatagcaat 6120
agttgtgtgg tccatagtaa tcatagaata taggaaaata ttaagacaaa gaaaaataga 6180
caggttaatt gatagactaa tagaaagagc agaagacagt ggcaatgaga gtgaaggaga 6240
aatatcagca cttgtggaga tgggggtgga gatggggcac catgctcctt gggatgttga 6300
tgatctgtag tgctacagaa aaattgtggg tcacagtcta ttatggggta cctgtgtgga 6360
aggaagcaac caccactcta ttttgtgcat cagatgctaa agcatatgat acagaggtac 6420
ataatgtttg ggccacacat gcctgtgtac ccacagaccc caacccacaa gaagtagtat 6480
tggtaaatgt gacagaaaat tttaacatgt ggaaaaatga catggtagaa cagatgcatg 6540
aggatataat cagtttatgg gatcaaagcc taaagccatg tgtaaaatta accccactct 6600
gtgttagttt aaagtgcact gatttgaaga atgatactaa taccaatagt agtagcggga 6660
gaatgataat ggagaaagga gagataaaaa actgctcttt caatatcagc acaagcataa 6720
gaggtaaggt gcagaaagaa tatgcatttt tttataaact tgatataata ccaatagata 6780
atgatactac cagctataag ttgacaagtt gtaacacctc agtcattaca caggcctgtc 6840
caaaggtatc ctttgagcca attcccatac attattgtgc cccggctggt tttgcgattc 6900
taaaatgtaa taataagacg ttcaatggaa caggaccatg tacaaatgtc agcacagtac 6960
aatgtacaca tggaattagg ccagtagtat caactcaact gctgttaaat ggcagtctag 7020
cagaagaaga ggtagtaatt agatctgtca atttcacgga caatgctaaa accataatag 7080

tacagctgaa cacatctgta gaaattaatt gtacaagacc caacaacaat acaagaaaaa 7140
gaatccgtat ccagagagga ccagggagag catttgttac aataggaaaa ataggaaata 7200
tgagacaagc acattgtaac attagtagag caaaatggaa taacacttta aaacagatag 7260
ctagcaaatt aagagaacaa tttggaaata ataaaacaat aatctttaag caatcctcag 7320
gaggggaccc agaaattgta acgcacagtt ttaattgtgg aggggaattt ttctactgta 7380
attcaacaca actgtttaat agtacttggt ttaatagtac ttggagtact gaagggtcaa 7440
ataacactga aggaagtgac acaatcaccc tcccatgcag aataaaacaa attataaaca 7500
tgtggcagaa agtaggaaaa gcaatgtatg cccctcccat cagtggacaa attagatgtt 7560
catcaaatat tacagggctg ctattaacaa gagatggtgg taatagcaac aatgagtccg 7620
agatcttcag acctggagga ggagatatga gggacaattg gagaagtgaa ttatataaat 7680
ataaagtagt aaaaattgaa ccattaggag tagcacccac caaggcaaag agaagagtgg 7740
tgcagagaga aaaaagagca gtgggaatag gagctttgtt ccttgggttc ttgggagcag 7800
caggaagcac tatgggcgca gcctcaatga cgctgacggt acaggccaga caattattgt 7860
ctggtatagt gcagcagcag aacaatttgc tgagggctat tgaggcgcaa cagcatctgt 7920
tgcaactcac agtctggggc atcaagcagc tccaggcaag aatcctggct gtggaaagat 7980
acctaaagga tcaacagctc ctggggattt ggggttgctc tggaaaactc atttgcacca 8040
ctgctgtgcc ttggaatgct agttggagta ataaatctct ggaacagatt tggaatcaca 8100
cgacctggat ggagtgggac agagaaatta acaattacac aagcttaata cactccttaa 8160
ttgaagaatc gcaaaaccag caagaaaaga atgaacaaga attattggaa ttagataaat 8220
gggcaagttt gtggaattgg tttaacataa caaattggct gtggtatata aaattattca 8280
taatgatagt aggaggcttg gtaggtttaa gaatagtttt tgctgtactt tctatagtga 8340
atagagttag gcagggatat tcaccattat cgtttcagac ccacctccca accccgaggg 8400
gacccgacag gcccgaagga atagaagaag aaggtggaga gagagacaga gacagatcca 8460
ttcgattagt gaacggatcc ttggcactta tctgggacga tctgcggagc ctgtgcctct 8520
tcagctacca ccgcttgaga gacttactct tgattgtaac gaggattgtg gaacttctgg 8580
gacgcagggg gtgggaagcc ctcaaatatt ggtggaatct cctacagtat tggagtcagg 8640
aactaaagaa tagtgctgtt agcttgctca atgccacagc catagcagta gctgagggga 8700
cagatagggt tatagaagta gtacaaggag cttgtagagc tattcgccac atacctagaa 8760
gaataagaca gggcttggaa aggattttgc tataagatgg gtggcaagtg gtcaaaaagt 8820
agtgtgattg gatggcctac tgtaagggaa agaatgagac gagctgagcc agcagcagat 8880
agggtgggag cagcatctcg agacctggaa aaacatggag caatcacaag tagcaataca 8940
gcagctacca atgctgcttg tgcctggcta gaagcacaag aggaggagga ggtgggtttt 9000
ccagtcacac ctcaggtacc tttaagacca atgacttaca aggcagctgt agatcttagc 9060
cactttttaa aagaaaaggg gggactggaa gggctaattc actcccaaag aagacaagat 9120
atccttgatc tgtggatcta ccacacacaa ggctacttcc ctgattagca gaactacaca 9180

ccagggccag gggtcagata tccactgacc tttggatggt gctacaagct agtaccagtt 9240
gagccagata agatagaaga ggccaataaa ggagagaaca ccagcttgtt acaccctgtg 9300
agcctgcatg ggatggatga cccggagaga gaagtgttag agtggaggtt tgacagccgc 9360
ctagcatttc atcacgtggc ccgagagctg catccggagt acttcaagaa ctgctgacat 9420
cgagcttgct acaagggact ttccgctggg gactttccag ggaggcgtgg cctgggcggg 9480
actggggagt ggcgagccct cagatcctgc atataagcag ctgctttttg cctgtactgg 9540
gtctctctgg ttagaccaga tctgagcctg ggagctctct ggctaactag ggaacccact 9600
gcttaagcct caataaagct tgccttgagt gcttcaagta gtgtgtgccc gtctgttgtg 9660
tgactctggt aactagagat ccctcagacc cttttagtca gtgtggaaaa tctctagca 9719

<210> 5
<211> 1998
<212> DNA
<213> Homo sapiens

<400> 5
cttccaagct ggttttgaag tcgcggccgc tgttcctggg acgtccggtt gaccgcgcgt 60
ctgctgcaga gaccatgtct gccgacgggg cagaggctga tggcagcacc caggtgacag 120
tggaagaacc ggtacagcag cccagtgtgg tggaccgtgt ggccagcatg cctctgatca 180
gctccacctg cgacatggtg tccgcagcct atgcctccac caaggagagc tacccgcacg 240
tcaagactgt ctgcgacgca gcagagaagg gagtgaggac cctcacggcg gctgctgtca 300
gcggggctca gccgatcctc tccaagctgg agccccagat tgcatcagcc agcgaatacg 360
cccacagggg gctggacaag ttggaggaga acctccccat cctgcagcag cccacggaga 420
aggtcctggc ggacaccaag gagcttgtgt cgtctaaggt gtcgggggcc caagagatgg 480
tgtctagcgc caaggacacg gtggccaccc aattgtcgga ggcggtggac gcgacccgcg 540
gtgctgtgca gagcggcgtg gacaagacaa agtccgtagt gaccggcggc gtccaatcag 600
tcatgggctc ccgcttgggc cagatggtgc tgagtggggt cgacacggtg ctggggaagt 660
cggaggagtg ggcggacaac cacctgcccc ttacggatgc cgaactggcc cgcatcgcca 720
catccctgga tggcttcgac gtcgcgtccg tgcagcagca gcggcaggaa cagagctact 780
tcgtacgtct gggctccctg tcggagaggc tgcggcagca cgcctatgag cactcgctgg 840
gcaagcttcg agccaccaag cagagggcac aggaggctct gctgcagctg tcgcaggccc 900
taagcctgat ggaaactgtc aagcaaggcg ttgatcagaa gctggtggaa ggccaggaga 960
agctgcacca gatgtggctc agctggaacc agaagcagct ccagggcccc gagaaggagc 1020
cgcccaagcc agagcaggtc gagtcccggg cgctcaccat gttccgggac attgcccagc 1080
aactgcaggc cacctgtacc tccctggggt ccagcattca gggcctcccc accaatgtga 1140

aggaccaggt gcagcaggcc cgccgccagg tggaggacct ccaggccacg ttttccagca 1200
tccactcctt ccaggacctg tccagcagca ttctggccca gagccgtgag cgtgtcgcca 1260
gcgcccgcga ggccctggac cacatggtgg aatatgtggc ccagaacaca cctgtcacgt 1320
ggctcgtggg accctttgcc cctggaatca ctgagaaagc cccggaggag aagaaatagg 1380
gggagaggag aggactcagc gggccccgtc tctataatgc agctgtgctc tggagtcctc 1440
aacccggggc tcatttcaaa cttattttct acccactcct cccagctctt ctgtgctgtc 1500
cacttgggaa gctaaggctc tcaaaacggg catcacccag ttgacccatc tctcagcctc 1560
tctgagcttg gaagaagcct gttctgagcc tcaccctatc agtcagtaga gagagatgtc 1620
cagaaaaaat atctttcagg aaagttctcc cctgcagaat tttttttcct tgttaaatat 1680
caggaatata ggccgggtgc ggtggctcac acctgtaatc ccagcacttt gggaggctga 1740
ggcgggcgga acacctgagg tcaggtgttc gagaccagcc aggccaacat ggtgaaaccc 1800
cgtctctact aaaaatacaa aaaaaaaatg agccgggcat ggtagcaggt gtctgttatc 1860
ccagttagga ggctgaggca agagaatctc ttgaacctga gaggcggagg ttgcagtgag 1920
ccaagatcgc gccattgcac tccagcctgg gggacaagag tgagacttag tctcaaaaaa 1980
aaaaaaaaaa aaaaaaaa 1998

<210> 6
<211> 12
<212> PRT
<213> Human immunodeficiency virus type 1

<220>
<221> MISC_FEATURE
<222> (2)..(2)
<223> X means Val

<220>
<221> MISC_FEATURE
<222> (2)..(2)
<223> X means Leu

<220>
<221> MISC_FEATURE
<222> (2)..(2)
<223> X means Ile

<220>
<221>  MISC_FEATURE
<222>  (2)..(2)
<223>  X means Thr

<220>
<221>  MISC_FEATURE
<222>  (2)..(2)
<223>  X means Arg

<220>
<221>  MISC_FEATURE
<222>  (2)..(2)
<223>  X means Glu

<220>
<221>  MISC_FEATURE
<222>  (4)..(4)
<223>  X means Ser

<220>
<221>  MISC_FEATURE
<222>  (4)..(4)
<223>  X means Thr

<220>
<221>  MISC_FEATURE
<222>  (4)..(4)
<223>  X means Arg

<220>
<221>  MISC_FEATURE
<222>  (4)..(4)
<223>  X means Ile

<220>
<221>  MISC_FEATURE
<222>  (4)..(4)
<223>  X means Lys

<220>
<221>  MISC_FEATURE
<222>  (5)..(5)
<223>  X means Gly

<220>
<221>  MISC_FEATURE
<222>  (5)..(5)
<223>  X means Ala

<220>
<221>  MISC_FEATURE
<222>  (6)..(6)
<223>  X means Gly

<220>
<221>  MISC_FEATURE
<222>  (6)..(6)
<223>  X means Arg

<220>
<221>  MISC_FEATURE
<222>  (6)..(6)
<223>  X means Lys

<220>
<221>  MISC_FEATURE
<222>  (6)..(6)

<223>  X means Glu

<220>
<221>  MISC_FEATURE
<222>  (6)..(6)
<223>  X means Ser

<220>
<221>  MISC_FEATURE
<222>  (6)..(6)
<223>  X means Thr

<220>
<221>  MISC_FEATURE
<222>  (6)..(6)
<223>  X means Lys

<220>
<221>  MISC_FEATURE
<222>  (6)..(6)
<223>  X means Asp

<220>
<221>  MISC_FEATURE
<222>  (7)..(7)
<223>  X means Glu

<220>
<221>  MISC_FEATURE
<222>  (7)..(7)
<223>  X means Lys

<220>
<221>  MISC_FEATURE
<222>  (7)..(7)

<223>  X means Arg

<220>
<221>  MISC_FEATURE
<222>  (7)..(7)
<223>  X means Gln

<220>
<221>  MISC_FEATURE
<222>  (7)..(7)
<223>  X means Gly

<220>
<221>  MISC_FEATURE
<222>  (8)..(8)
<223>  X means Leu

<220>
<221>  MISC_FEATURE
<222>  (8)..(8)
<223>  X means Phe

<220>
<221>  MISC_FEATURE
<222>  (8)..(8)
<223>  X means Lys

<220>
<221>  MISC_FEATURE
<222>  (8)..(8)
<223>  X means Gln

<220>
<221>  MISC_FEATURE

<222>  (9)..(9)
<223>  X means Asp

<220>
<221>  MISC_FEATURE
<222>  (9)..(9)
<223>  X means Glu

<220>
<221>  MISC_FEATURE
<222>  (10)..(10)
<223>  X means Arg

<220>
<221>  MISC_FEATURE
<222>  (10)..(10)
<223>  X means Ala

<220>
<221>  MISC_FEATURE
<222>  (10)..(10)
<223>  X means Ser

<220>
<221>  MISC_FEATURE
<222>  (10)..(10)
<223>  X means Lys

<220>
<221>  MISC_FEATURE
<222>  (10)..(10)
<223>  X means Gln

<220>
<221>  MISC_FEATURE

<222>  (10)..(10)

<223>  X means Thr


<220>

<221>  MISC_FEATURE

<222>  (10)..(10)

<223>  X means Glu


<220>

<221>  MISC_FEATURE

<222>  (10)..(10)

<223>  X means Asn


<220>

<221>  MISC_FEATURE

<222>  (10)..(10)

<223>  X means Asp


<220>

<221>  MISC_FEATURE

<222>  (10)..(10)

<223>  X means Pro


<400>  6

Ser Xaa Leu Xaa Xaa Xaa Xaa Xaa Xaa Xaa Trp Glu
1               5                   10

<210>  7

<211>  23

<212>  PRT

<213>  Human immunodeficiency virus type 1


<220>

<221>  MISC_FEATURE

<222>  (1)..(1)

<223>  X means Met

<220>
<221>  MISC_FEATURE
<222>  (1)..(1)
<223>  X means Leu

<220>
<221>  MISC_FEATURE
<222>  (1)..(1)
<223>  X means Val

<220>
<221>  MISC_FEATURE
<222>  (1)..(1)
<223>  X means Ile

<220>
<221>  MISC_FEATURE
<222>  (2)..(2)
<223>  X means Gly

<220>
<221>  MISC_FEATURE
<222>  (2)..(2)
<223>  X means Ser

<220>
<221>  MISC_FEATURE
<222>  (2)..(2)
<223>  X means Thr

<220>
<221>  MISC_FEATURE
<222>  (4)..(4)

<223>  X means Arg

<220>
<221>  MISC_FEATURE
<222>  (4)..(4)
<223>  X means Gly

<220>
<221>  MISC_FEATURE
<222>  (4)..(4)
<223>  X means Ser

<220>
<221>  MISC_FEATURE
<222>  (4)..(4)
<223>  X means Lys

<220>
<221>  MISC_FEATURE
<222>  (7)..(7)
<223>  X means Val

<220>
<221>  MISC_FEATURE
<222>  (7)..(7)
<223>  X means Leu

<220>
<221>  MISC_FEATURE
<222>  (7)..(7)
<223>  X means Ile

<220>
<221>  MISC_FEATURE

<222>  (7)..(7)
<223>  X means Thr

<220>
<221>  MISC_FEATURE
<222>  (7)..(7)
<223>  X means Arg

<220>
<221>  MISC_FEATURE
<222>  (7)..(7)
<223>  X means Glu

<220>
<221>  MISC_FEATURE
<222>  (9)..(9)
<223>  X means Ser

<220>
<221>  MISC_FEATURE
<222>  (9)..(9)
<223>  X means Thr

<220>
<221>  MISC_FEATURE
<222>  (9)..(9)
<223>  X means Arg

<220>
<221>  MISC_FEATURE
<222>  (9)..(9)
<223>  X means Ile

<220>
<221>  MISC_FEATURE

<222>  (9)..(9)
<223>  X means Lys

<220>
<221>  MISC_FEATURE
<222>  (10)..(10)
<223>  X means Gly

<220>
<221>  MISC_FEATURE
<222>  (10)..(10)
<223>  X means Ala

<220>
<221>  MISC_FEATURE
<222>  (11)..(11)
<223>  X means Gly

<220>
<221>  MISC_FEATURE
<222>  (11)..(11)
<223>  X means Arg

<220>
<221>  MISC_FEATURE
<222>  (11)..(11)
<223>  X means Lys

<220>
<221>  MISC_FEATURE
<222>  (11)..(11)
<223>  X means Glu

<220>

<221>  MISC_FEATURE
<222>  (11)..(11)
<223>  X means Ser

<220>
<221>  MISC_FEATURE
<222>  (11)..(11)
<223>  X means Thr

<220>
<221>  MISC_FEATURE
<222>  (11)..(11)
<223>  X means Lys

<220>
<221>  MISC_FEATURE
<222>  (11)..(11)
<223>  X means Asp

<220>
<221>  MISC_FEATURE
<222>  (12)..(12)
<223>  X means Glu

<220>
<221>  MISC_FEATURE
<222>  (12)..(12)
<223>  X means Lys

<220>
<221>  MISC_FEATURE
<222>  (12)..(12)
<223>  X means Arg

<220>

<221>  MISC_FEATURE
<222>  (12)..(12)
<223>  X means Gln

<220>
<221>  MISC_FEATURE
<222>  (12)..(12)
<223>  X means Gly

<220>
<221>  MISC_FEATURE
<222>  (13)..(13)
<223>  X means Leu

<220>
<221>  MISC_FEATURE
<222>  (13)..(13)
<223>  X means Phe

<220>
<221>  MISC_FEATURE
<222>  (13)..(13)
<223>  X means Lys

<220>
<221>  MISC_FEATURE
<222>  (13)..(13)
<223>  X means Gln

<220>
<221>  MISC_FEATURE
<222>  (14)..(14)
<223>  X means Asp

<220>
<221> MISC_FEATURE
<222> (14)..(14)
<223> X means Glu

<220>
<221> MISC_FEATURE
<222> (15)..(15)
<223> X means Arg

<220>
<221> MISC_FEATURE
<222> (15)..(15)
<223> X means Ala

<220>
<221> MISC_FEATURE
<222> (15)..(15)
<223> X means Ser

<220>
<221> MISC_FEATURE
<222> (15)..(15)
<223> X means Lys

<220>
<221> MISC_FEATURE
<222> (15)..(15)
<223> X means Gln

<220>
<221> MISC_FEATURE
<222> (15)..(15)
<223> X means Thr

<220>
<221>  MISC_FEATURE
<222>  (15)..(15)
<223>  X means Glu

<220>
<221>  MISC_FEATURE
<222>  (15)..(15)
<223>  X means Asn

<220>
<221>  MISC_FEATURE
<222>  (15)..(15)
<223>  X means Asp

<220>
<221>  MISC_FEATURE
<222>  (15)..(15)
<223>  X means Pro

<220>
<221>  MISC_FEATURE
<222>  (18)..(18)
<223>  X means Lys

<220>
<221>  MISC_FEATURE
<222>  (18)..(18)
<223>  X means Arg

<220>
<221>  MISC_FEATURE
<222>  (18)..(18)
<223>  X means Asn

<220>
<221> MISC_FEATURE
<222> (18)..(18)
<223> X means Glu

<220>
<221> MISC_FEATURE
<222> (18)..(18)
<223> X means Ala

<220>
<221> MISC_FEATURE
<222> (18)..(18)
<223> X means Ser

<220>
<221> MISC_FEATURE
<222> (19)..(19)
<223> X means Ile

<220>
<221> MISC_FEATURE
<222> (19)..(19)
<223> X means Met

<220>
<221> MISC_FEATURE
<222> (19)..(19)
<223> X means Gln

<220>
<221> MISC_FEATURE
<222> (19)..(19)
<223> X means Thr

<220>
<221>  MISC_FEATURE
<222>  (20)..(20)
<223>  X means Arg

<220>
<221>  MISC_FEATURE
<222>  (20)..(20)
<223>  X means Lys

<220>
<221>  MISC_FEATURE
<222>  (20)..(20)
<223>  X means Trp

<220>
<221>  MISC_FEATURE
<222>  (20)..(20)
<223>  X means Gln

<220>
<221>  MISC_FEATURE
<222>  (20)..(20)
<223>  X means His

<220>
<221>  MISC_FEATURE
<222>  (20)..(20)
<223>  X means Tyr

<220>
<221>  MISC_FEATURE
<222>  (21)..(21)

<223>  X means Leu

<220>
<221>  MISC_FEATURE
<222>  (21)..(21)
<223>  X means Ser

<220>
<221>  MISC_FEATURE
<222>  (22)..(22)
<223>  X means Arg

<220>
<221>  MISC_FEATURE
<222>  (22)..(22)
<223>  X means Lys

<400>  7

Xaa Xaa Ala Xaa Ala Ser Xaa Leu Xaa Xaa Xaa Xaa Xaa Xaa Xaa Trp
1               5                   10                  15

Glu Xaa Xaa Xaa Xaa Xaa Pro
            20